# EUROPEAN PATENT SPECIFICATION

(11) **EP 4 391 862 B1**
(45) Date of publication and mention of the grant of the patent: **04.11.2026**
(21) Application number: 21769338.1
(22) Date of filing: 24.08.2021
(51) Int. Cl.: A61K 8/26, A61Q 5/12, A45D 19/02, A45D 40/24, B65D 35/22, A61K 8/23

(54) **PACKAGE FOR DISPENSING DUAL-PHASE COSMETIC COMPOSITION**
VERPACKUNG ZUR ABGABE EINER ZWEIPHASIGEN KOSMETISCHEN ZUSAMMENSETZUNG
EMBALLAGE POUR LA DISTRIBUTION D'UNE COMPOSITION COSMÉTIQUE À DEUX PHASES

(43) Date of publication of application: 03.07.2024
(73) Proprietor: The Procter & Gamble Company, Cincinnati, OH 45202 (US)
(72) Inventor: CHUA, Michele Tsu Fern, Singapor 138547 (SG); GOH, Chern Ping, Singapor 138547 (SG); HE, Rong, Beijing 101312 (CN); LI, Qiao, Beijing 101312 (CN); TAY JING WEN, Marcus, Singapor 138547 (SG); LEE, Martin Chang Shou, Singapor 138547 (SG); ZHANG, Jun Ji, Singapor 138547 (SG)
(74) Representative: P&G Patent Germany
(86) International application number: PCT/CN2021/114220
(87) International publication number: WO 2023/023927

(56) References cited:
- EP-B1- 1 718 541
- WO-A1-02/19977
- KR-A- 20200 122 083
- US-A1- 2008 128 425

## Description

### FIELD OF THE INVENTION

A package for a dual-phase cosmetic composition, in particular a dual-phase rinse-off hair treatment composition that provides a warming sensation and hair conditioning is provided herein.

### BACKGROUND OF THE INVENTION

A variety of hair treatment products are available to consumers that include leave-on-treatments and rinse-off-treatments. In general, hair treatments are used to improve the feel, appearance, and manageability of hair. Some consumers may want a hair treatment that not only provides excellent hair conditioning but also provides a pleasant user experience, such as a warming sensation when rubbed in a user's hands and/or when applied to a user's hair and/or scalp.

One way to provide both excellent conditioning and a warming sensation is to use a dual-phase rinse-off hair treatment composition. However, it can be difficult to store and dispense dual-phase compositions. First, dual-phase compositions often need to be stored separately, for example in multi-chamber packages, to prevent the active ingredients in each composition from interacting with one another. Second, consumers generally prefer when the compositions dispense evenly, so both compositions are used up at approximately the same time, leaving little residue remaining in the package. This can be difficult because each composition has a different formula and viscosity that can cause them to dispense at different rates. Third, it can be important for the user experience if the dispensed product has a pleasing appearance in the user's palm.

WO 02/19977 A1 is related to hair care kits used for warming a hair care composition before the composition is applied to the hair.

KR 2020/0122083 A relates to dual tubes for cosmetics and a cosmetic product using the same.

EP 1 718 541 B1 refers to a multi-chamber tube, especially a dual-chamber tube, which comprises an outer tube pipe having a tube neck sitting on a tube shoulder and leading to an outlet. A first chamber can be filled with a first preparation and a second chamber with a second preparation. The tube neck is provided with a first passage channel for the passage of the first preparation and with a second passage channel for the passage of the second preparation. The passage channels lead to the outlet where the first preparation and the second preparation meet to form a common strand.

US 2008/128425 A1 sets out a dual warming and analgesic composition comprising a hydrous portion comprising water and an anhydrous portion comprising a zeolite a carrier and a suspending or dispersing agent, wherein the anhydrous portion and the hydrous portion are combined in use to provide warming and analgesic effects without the addition of external water.

Therefore, there is a need for a package for a dual-phase hair treatment composition that separates the phases during storage and evenly dispenses a composition with a pleasing appearance.

### SUMMARY OF THE INVENTION

A rinse-off hair treatment product comprising: (a) a tube package comprising: (i) an inner tube comprising an inner tube wall and an inner chamber; (ii) an outer tube comprising an outer tube wall an outer chamber formed between the outer tube wall and the inner tube wall; (iii) one or more outer orifices fluidly connected to the outer chamber by one or more outer nozzle channels; (iv) one or more central orifices fluidly connected to the inner chamber by one or more inner nozzle channels; wherein the ratio of the area of the one or more central orifices to the one or more outer orifices is from 2 to 6, preferably from 2.5 to 5.5, more preferably from 3 to 5, and even more preferably from 3.5 to 4.5; (b) a dual-phase hair treatment composition comprising: (i) a warming composition having comprising a viscosity; (ii) a conditioning composition comprising a viscosity; wherein the viscosity of the warming composition is greater than the viscosity of the conditioning composition; wherein inner chamber contains the warming composition; wherein the outer chamber contains the conditioning composition; wherein the warming composition and the conditioning composition are physically separated within the tube package.

### BRIEF DESCRIPTION OF THE DRAWINGS

The patent or application file contains at least one drawing executed in color. Copies of this patent or patent application publication with color drawing(s) will be provided by the Office upon request and payment of the necessary fee.

While the specification concludes with claims particularly pointing out and distinctly claiming the subject matter as described herein, it is believed that the invention can be more readily understood from the following description taken in connection with the accompanying drawings, in which:
FIG. 1A is a perspective view of a tube package for dispensing a hair treatment composition;
FIG. 1B is a perspective view of a tube package for dispensing a hair treatment composition with a cap;
FIG. 1C is a cross-sectional view along axis-Y of the tube package with the cap of FIG. 1B;
FIG. 2 is a table showing the orifice design and the dispensed composition for Examples 1-4;
FIG. 3 is a table showing the orifice design and the dispensed composition for Examples 5-8;
FIG. 4A is a table showing the orifice design and the dispensed composition for Examples 9-11; and
FIG. 4B is a table showing the orifice design and the dispensed composition for Examples 12-14.

### DETAILED DESCRIPTION OF THE INVENTION

Two-chamber or multi-chamber packages are commonly used for keeping compositions separate prior to dispensing. One example of a two-chamber package is where one tube is inserted into another, the nozzle at the top of the inner tube here having been inserted within the nozzle channel of the outer tube. The end of the two tubes of this "tube-in-tube package" can be joined by a snap fit plug seal. The two tubes define an interior and an exterior chamber, and these chambers lead to the shared top region or shared discharge region. In another example, a separating wall separates a tube in the form of flexible tube material into two adjacent chambers ("side-by-side"). Like the tube-in-tube package, the nozzle channels in this design do not coalesce until they reach the orifice region at the end of the tube neck. In both the tube-in-tube and side-by-side packages the compositions are discharged from the packages as soon as pressure is applied to the package.

For compositions that must be stored separately, like dual-phase hair treatment compositions that can provide a warming sensation and conditioning, current multi-chamber packages do not dispense a consumer acceptable product. Since each hair treatment composition has a unique formula and viscosity, current dual-phase packaging does not dispense the product evenly, resulting in a poor user experience because the product does not provide the proper appearance, warming sensation, and/or conditioning performance and there can be excessive composition wasted in the package after one composition is used up. Further, when both compositions are visible in the expelled strand, it not only appears more pleasing and fun to a user, but it was found that users also intuitively mix the product together, which immediately causes the warming sensation and an improved consumer experience.

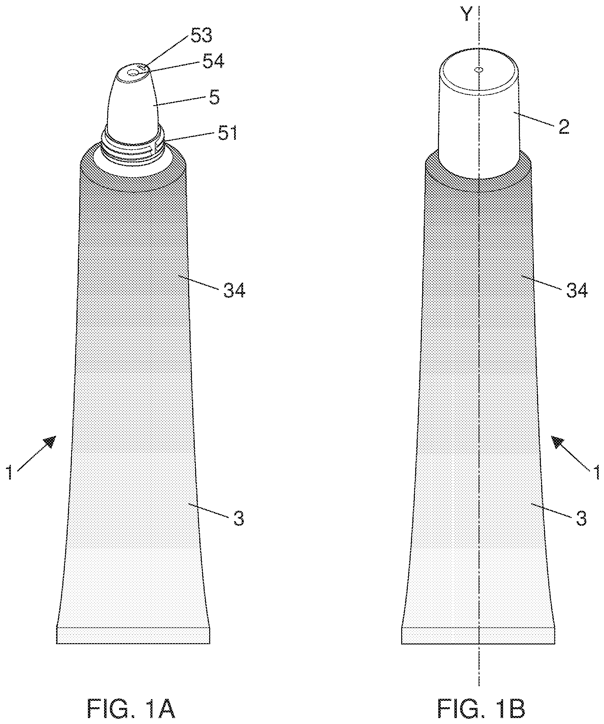

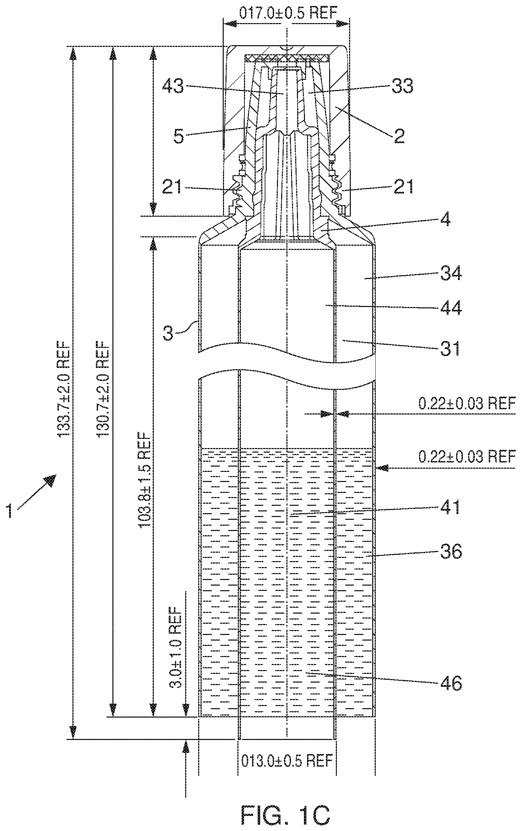

FIGS. 1A, 1B, and 1C shows a tube package 1 that is formed in a "tube-in-tube" manner for storing two hair treatment compositions. Tube package 1 can store two hair treatment compositions in a shared package in chambers that are separate from one another. The mixing or the confluence of the two compositions does not occur until the product is used. The two-chamber tube package can include outer tube 3 and inner tube 4. Outer tube 3 has an outer chamber 31 that is formed between the outer tube wall 34 and inner tube wall 44. Outer chamber 31 can be adapted to store and dispense a conditioner composition 36. Outer chamber 31 coaxially surrounds a pipe-like inner tube 4 with inner tube walls 44 that form an inner chamber 41 adapted for storing and dispensing a warming composition 46.

The two chambers 31 and 41 have different volumes that can be in a certain prescribed ratio to one another. It was determined that in order to have the best product performance with the least amount of wasted product, when full (e.g. at the time of purchase, which can optionally include a headspace in the inner or outer chamber) the conditioning composition 36 in the outer tube 4 can contain more product by weight than the warming composition in the inner tube and therefore the weight ratio of the conditioning composition to the warming composition can be greater than 1, alternatively greater than 1 to 5, alternatively from 1.1 to 4, alternatively from 1.2 to 2.5, alternatively from 1.25 to 2.25, alternatively from 1.3 to 2, alternatively from 1.35 to 1.9, alternatively from 1.4 to 1.8, and alternatively from 1.5 to 1.75.

The ratio of the weight of the dispensed composition in the outer tube to the weight of the dispensed composition in the inner tube can be greater than 1, alternatively from 1 to 3, alternatively from 1.1 to 2, alternatively from 1.2 to 1.75, alternatively from 1.3 to 1.5, and alternatively 1.4.

Nozzle 5 has external threads 51 that are adapted to engage with the internal threads 21 on cap 2 so the nozzle 5 and cap 2 can be screwed together.

Nozzle 5 can include at least central orifice 54, which is in fluid communication with inner nozzle channel 43 and inner chamber 41, and outer orifice 53, which is in fluid communication with one or more outer nozzle channels 33 and outer chamber 31. Central orifice 54 and the one or more outer orifice 53 can be any shape. In some examples, central orifice 54 can be a circle or oval and the one or more outer orifices can be straight slots, curved slots, half-circles, a circular triangle, or combinations thereof. In some examples, the central orifice can be one orifice and in other examples the central orifice can have more than one orifice. In some examples, the outer orifice can be a single orifice, alternatively the outer orifice can be two or three orifices, and alternatively the outer orifice can be two or more orifices.

The area of the central orifice can be larger than the area of the two or more outer orifices. The ratio of the area of the central orifice to the outer orifice can be greater than or equal to 1:1, alternatively from 1 to 10, alternatively from 1.3 to 7, alternatively from 2 to 6, alternatively from 2.5 to 5.5, alternatively from 3 to 5, alternatively from 3.5 to 4.5, and alternatively 4.

The central orifice can comprise one or more orifices. In some examples, the central orifice comprises only one orifice. The central orifice can have an area of from 2 mm² to 9 mm², alternatively from 3 mm² to 7 mm², alternatively from 4 mm² to 6 mm², and alternatively from 4.5 mm² to 5.5 mm².

The outer orifice can comprise one or more orifices. In some examples, the outer orifice comprises only one orifice. The outer orifice can have an area of from 0.25 mm² ₜo 5 mm², alternatively from 0.5 mm² to 3.5 mm², alternatively from 0.75 mm² to 2.5 mm², and alternatively from 1 mm² to 1.5 mm².

The one or more outer nozzle channels 33 can be distinct from inner nozzle channel 43, thus, when pressure is applied to the package, in particular when pressure is applied to outer tube wall 34, conditioner composition 36 and warming composition 46 can be initially conveyed separately until conditioner composition 36 exits outer orifice 53 and warming composition 46 exits central orifice 54 in a shared strand into a user's palm and/or onto a cleaning implement. In this example, outer tube 3 and inner tube 4 can be made from a flexible material, for example a recyclable laminated film material made from an aluminum barrier and/or polymers, and both the outer tube 3 and the inner tube 4 can be sealed at the rear, opposite the nozzle, by any suitable means, for example a joint that is heat sealed or crimped. In some examples, the inner and outer tube can be made of the same laminate material. In other examples, the inner and outer tube can be made of different materials. It was found that not only did consumers prefer packaging made from a material that is relatively soft, which makes the package easy to squeeze and easy to dispense the hair treatment composition, but it also made the warming sensation of the composition more noticeable. In another example, the outer tube wall can be a laminate structure that can include a barrier, such as aluminum, ethylene vinyl alcohol, or combinations thereof. The outer tube wall and the inner tube wall can be made out of the same material(s) and have the same thickness and same structure. Alternatively, the outer tube wall and the inner tube wall can have different thicknesses, different materials, and/or a different structure. In another example, the outer tube wall and the inner tube wall are substantially free of or free of high-density polyethylene. The tube package can be recyclable.

The outer tube 3 can have a body with a stiffness of from 3.0 N to 9.5 N, alternatively from 3.0 N to 5.5 N, alternatively from 3.2 N to 4.0 N and a wall thickness of from 300 µm to 600 µm, from 300 µm to 500 µm, and from 300 µm to 400 µm. The inner tube 4 can have a body with a stiffness of from 2.9 N to 9.5 N, alternatively from 3.0 N to 6.0 N, and alternatively from 4.0 N to 5.0 N and a wall thickness of from 200 µm to 600 µm from 300 µm to 500 µm, from 400 µm to 500 µm. The dual-phase composition can warm instantly when mixed, and the composition can quickly absorb and penetrate to the hair core providing excellent overall conditioning. The dual-phase composition can repair damaged hair and can leave hair feeling healthy and strong from the core. In addition, the dual-phase conditioner can improve hair texture, leave hair looking shiny and healthy, improves hair volume and/or fullness, prevents hair breakage, keeps hair smooth and soft longer as compared to traditional single-phase hair conditioners, nourishes and/or heals hair from the inside and/or core, and improves hair quality with each use. The dual phase composition can heal hair from the core and can make hair resilient and strong from the core.

The tube package can be packaged in a secondary package. In some examples, the secondary carton can be a pulp-based carton. The secondary package can contain any suitable number of tube packages. In one example the secondary package can contain a single tube package, alternatively 2-3 tubes, alternatively 2-5 tubes, alternatively 3-7 tubes, alternatively 3-10 tubes, and alternatively 4-12 tubes. In one example the secondary package can contain a plurality of tubes, for example 7 tubes, and the user can get improved benefits with continued use. For example, using one tube can help smooth hair, three tubes can improve shine, and hair strength can be improved if seven tubes are used.

In some examples, which do not form part of the present invention, the outer tube can contain the warming composition and the inner tube can contain the conditioning composition and the weight ratios, dispensing ratios, and orifice ratios described above can remain the same or they can be inversed.

All percentages, parts and ratios are based upon the total weight of the compositions as described herein, unless otherwise specified. All such weights as they pertain to listed ingredients are based on the active level and, therefore, do not include carriers or by-products that may be included in commercially available materials.

The term "molecular weight" or "M.Wt." as used herein refers to the weight average molecular weight unless otherwise stated. The weight average molecular weight may be measured by gel permeation chromatography.

The cosmetic composition as described herein comprises a first composition and a second composition, wherein the first and second compositions are kept separate from one another until dispensed. The cosmetic composition is a rinse-off hair treatment composition. The first composition is a warming composition and can contain an inorganic heat generating agent and the second composition is a conditioning composition and can contain a cationic surfactant system comprising a mono-alkyl quaternized ammonium salt cationic surfactant, a high melting point fatty compound, and an aqueous carrier.

The warming composition has a greater viscosity than the viscosity of the conditioning composition. For example, the viscosity of the warming composition can be at least 1.25 times larger than the viscosity of the conditioning composition, alternatively 1.3 times larger, alternatively 1.4 times larger, alternatively 1.5 times larger, and alternatively 2 times larger. The viscosities can be measured on a 2.5 mL sample of the composition using a cone and plate Brookfield RS rheometer with cone C75-1 at constant shear rate of 2 s⁻¹, at 27°C at 3 mins.

The first composition and the second composition can be different colors, which can give the dispensed product stream a pleasing aesthetic appearance and can also prompt the user to intuitively mix the composition, thereby activating the warming sensation. Although kept separate until dispensing, the first composition and the second composition are put into contact upon dispensing into a user's palm.

### First Composition (Warming Composition)

One phase of the treatment composition can contain a warming composition that comprises an inorganic heat generating agent that generates heat by mixing with water. As the heating reaction does not begin until mixed with water, the carrier of the first composition can be anhydrous.

As used herein, "anhydrous" means that the compositions contain 5% or less of water, alternatively 3% or less, alternatively 1% or less, alternatively substantially free of water, and alternatively free of water. The anhydrous composition can warm to a temperature of, from 25°C to 80°C, alternatively from 30°C to 60°C, and alternatively from 35°C to 45°C. This temperature can be adjusted by, for example, choosing the heat generating agents, the amount of the heat generating agent, and additional agents that can control the heat generating reaction.

The inorganic heat generating agents that may be used, such as calcium sulfate, generally have an enthalpy change of -19.2 kJ mol⁻¹ and below. The inorganic heat generating agents useful herein may include, for example, chlorides, such as calcium chloride (CaCl₂, CaCl₂.H₂O, CaCl₂.2H₂O), magnesium chloride (MgCl₂, MgCl₂.2H₂O, MgCl₂.4H₂O), aluminum chloride (AlCl₃, AlCl₃.6H₂O), ferric chloride (FeCl₃, FeCl₃.2H₂O), and zinc chloride (ZnCl₂); sulfates such as magnesium sulfate (MgSO₄, MgSO₄.H₂O, MgSO₄.4H₂O), zinc sulfate (ZnSO₄.H₂O), ferrous sulfate (FeSO₄, FeSO₄.H₂O), and calcium sulfate (CaSO₄, CaSO₄.½H₂O, CaSO₄.H₂O); dry alum; calcium oxide (CaO); magnesium oxide (MgO); carbonates such as potassium carbonate (K₂CO₃) and sodium carbonate (Na₂CO₃); bromides such as magnesium bromide (MgBr₂), calcium bromide (CaBr₂) and aluminum bromide (AlBr₃); iodides such as magnesium iodide (MgI₂, MgI₂.6H₂O), calcium iodide (CaI₂) and aluminum iodide (AlI₃); zeolite; and sodium hydrogenophosphate (Na₂HPO₄). Some aspects may include anhydrous inorganic salts such as calcium sulfate (CaSO₄), magnesium sulfate (MgSO₄), calcium chloride (CaCl₂), magnesium chloride (MgCl₂), calcium oxide (CaO), and mixtures thereof, in view of their effective heat generation, mildness to hair and/or skin, and easy handling. Some aspects may include anhydrous magnesium sulfate (MgSO₄).

The inorganic heat generating agents useful herein may have an average diameter of, preferably from 0.01 um to 200 µm, more preferably from 0.05 µm to 30 µm, still more preferably from 0.1 µm to 20 µm, in view of preventing gritty feel.

The inorganic heat generating agent may be included in the compositions at a level by weight of the first composition of from 5% to 60%, or from 8% to 50%, or from 10% to 35%, from 10% to 30%, from 12% to 20%.

### Phase Changing Agents

The anhydrous cosmetic compositions can include a phase changing agent dispersed in an inert carrier. It is believed that the phase changing agent can have a certain melting point and can absorb heat from the heat generating agent by changing its phase from solid to liquid, and then, release the heat slowly by changing its phase from liquid to solid. Thus, it is believed that the phase changing agent can prevent the compositions from warming up to a higher temperature than expected, and provide prolonged warming from the compositions, without using coated heat generating agents.

The phase-changing agents as described herein can have a melting point of from 30°C to 70°C, preferably from 30 °C to 60 °C, more preferably from 35 °C to 50 °C. This melting point can be that of one kind of material. The melting point can also be a mixture of 2 or more kinds of materials, when 2 or more materials are miscible with each other. In this case, each material does not necessarily have a melting point of from 30°C to 70°C, however, mixtures thereof have a melting point of from 30°C to 70°C.

The phase-changing agents useful herein include, for example, amidoamines; fatty compounds such as fatty alcohols, fatty acids, fatty alcohol derivatives, fatty acid derivatives, and mixtures thereof; hydrocarbons such as solid paraffin; and mixtures thereof. Fatty compound useful herein are disclosed below under the title "High Melting Point Fatty Compound". Amidoamines useful herein are disclosed below under the title "Amidoamine". Preferred phase changing agents are fatty compounds such as fatty alcohols, fatty acids, fatty alcohol derivatives, fatty acid derivatives, and mixtures thereof; amidoamines; and mixtures thereof, in view of providing conditioning benefit. More preferred are fatty alcohols selected from the group consisting of cetyl alcohol (melting point = 46-55 °C), stearyl alcohol (melting point = 54-61 °C), and mixtures thereof, still more preferred are mixtures of cetyl alcohol and stearyl alcohol having a weight ratio of cetyl alcohol to stearyl alcohol of from 10:90 to 99:1 (melting point = from 48 °C to 58 °C). To function as a phase changing agent, materials can be dispersed, but not dissolved in an inert carrier. Materials can completely dissolve in an inert carrier, even if they have a melting point of from 30°C to 70°C, cannot function as the phase changing agents as described herein. For example, Japanese Patent Laid-open No. H11-228332 discloses a composition containing 5wt% of myristyl myristate having a melting point of from 41 to 43 °C and 65wt% of octyl stearate carrier (in Example 9), however, 5wt% of myristyl myristate is completely dissolved in 65wt% of octyl stearate carrier, thus, it cannot function as the phase changing agent as described herein.

In order to make phase changing agents dispersed in an inert carrier, materials which are insoluble in the inert carrier are used, or materials are contained at a higher level than its saturation point in the inert carrier. Materials having a lower solubility to an inert carrier can be used. The solubility depends on each combination of phase changing agents and inert carriers. For example, preferable combinations of phase changing agents and inert carriers can include; the combination of high melting point fatty compound as phase changing agent, and polyethylene glycol as an inert carrier; the combination of high melting point fatty compound as phase changing agent, and glycerin as an inert carrier; the combination of high melting point fatty compound as phase changing agent, and low melting point ester oils as an inert carrier; the combination of high melting point fatty compound as phase changing agent, and liquid paraffin as an inert carrier; the combination of hydrocarbons such as solid paraffin as phase changing agent, and polyethylene glycol as an inert carrier. Additional examples can include the combination of fatty alcohols selected from the group consisting of cetyl alcohol, stearyl alcohol, and mixtures thereof as phase changing agents, and polyethylene glycol as an inert carrier; the combination of fatty alcohols selected from the group consisting of cetyl alcohol, stearyl alcohol, and mixtures thereof as phase changing agents, and glycerin as an inert carrier; the combination of fatty alcohols selected from the group consisting of cetyl alcohol, stearyl alcohol, and mixtures thereof as phase changing agents, and pentaerythritol ester oils as an inert carrier. The phase changing agent can be included in the compositions at a level by weight of, preferably from 0.2% to 20%, more preferably from 0.5% to 15% still more preferably from 1 % to 10%.

### Polyoxyalkylene Derivatives

The anhydrous first warming composition may include polyoxyalkylene derivatives. It is believed that polyoxyalkylene derivatives can help the dispersion of inorganic heat generating agents in inert carriers, thus, prevent the agglomeration of inorganic heat generating agents which causes a gritty feel to the skin and/or hair. It is also believed that some of the polyoxyalkylene derivatives can provide a slippery feel, which eases the gritty feel caused by the inorganic heat generating agents.

The polyoxyalkylene derivatives useful herein can be water soluble polyoxyalkylene derivatives. The polyoxyalkylene derivatives useful herein may include, for example, polyoxyethylene/polyoxypropylene copolymer, polyoxyethylene alkyl ether, polyoxypropylene alkyl ether, polyoxyethylene alkyl ether ester, polyoxypropylene alkyl ether ester, polyoxyethylene glyceryl ester, polyoxypropylene glyceryl ester, and mixtures thereof. Among them, polyoxyethylene/polyoxypropylene copolymers may be used in view of preventing agglomeration of inorganic heat generating agents, and polyoxyethylene glyceryl esters may be used in view of providing slippery feel.

When the polyoxyalkylene derivative is used in view of preventing agglomeration of inorganic heat generating agents, the polyoxyalkylene derivative may be included in the compositions at a level by weight of, from 0.1% to 10%, alternatively from 0.5% to 10%, and alternatively from 1% to 5%. When the polyalkylene derivative is used in view of providing slippery feel, the polyoxyalkylene derivative may be included in the compositions at a level by weight of from 10% to 90%, alternatively from 15% to 85%, alternatively from 20% to 80%.

Polyoxyethylene alkyl ethers can include, for example, those of the formula RO(CH₂CH₂O)ₙH, wherein n is from 1 to 200, preferably from 20 to 100, and R is an alkyl having from 8 to 22 carbon atoms.

Polyoxyethylene glyceryl esters can include, for example, following (i) and (ii).
(i) PEG-Odified Glycerides having the Structure: 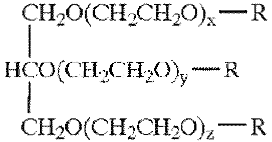 wherein one or more of the R groups is selected from saturated or unsaturated fatty acid moieties derived from animal or vegetable oils such as palmitic acid, lauric acid, oleic acid or linoleic acid wherein the fatty acid moieties have a carbon length chain of from 12 and 22, any other R groups are hydrogen, x, y, z are independently zero or more, the average sum of x+y+z (the degree of ethoxylation) is equal to from 10 to 45. The PEG-modified glycerides can have an HLB value of 20 or less, alternatively 15 or less, alternatively 11 or less. The PEG-modified glycerides can have from 2 to 3 fatty acid R groups, alternatively 3 fatty acid R groups (PEG-modified triglycerides). The average sum of x+y+z (the degree of ethoxylation) can equal to from 20 to 30, alternatively is an average sum of 5. PEG-substituted triglycerides having 3 oleic acid R groups, wherein the average degree of ethoxylation is 25 (PEG-25 glyceryl trioleate). Commercially available PEG-modified triglycerides can include Tagat^{®} TOO, Tegosoft^{®} GC, Tagat^{®} BL 276, (all three manufactured by Evonik Industries^{®} AG) and Crovol^{™} A-40, Crovol^{™} M-40 (manufactured by Croda^{®} Corporation). Other preferred commercially available PEG-modified triglycerides include Tagat^{®} S and Tagat^{®} S 2 (manufactured by Evonik Industries^{®} AG).
(ii) PEG-Modified Glyceryl Fatty Acid Esters having the Structure: 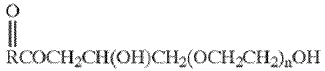 wherein n, the degree of ethoxylation, is from 4 to 200, alternatively from 5 to 150, alternatively from 20 to 120, and wherein R comprises an aliphatic radical having from 5 to 25 carbon atoms, alternatively from 7 to 20 carbon atoms. Suitable polyethylene glycol derivatives of glycerides can be polyethylene glycol derivatives of hydrogenated castor oil. For example, PEG-20 hydrogenated castor oil, PEG-30 hydrogenated castor oil, PEG-40 hydrogenated castor oil, PEG-45 hydrogenated castor oil, PEG-50 hydrogenated castor oil, PEG-54 hydrogenated castor oil, PEG-55 hydrogenated castor oil, PEG-60 hydrogenated castor oil, PEG-80 hydrogenated castor oil, and PEG-100 hydrogenated castor oil. In some examples, the composition can include PEG-60 hydrogenated castor oil. Other suitable polyethylene glycol derivatives of glycerides can be polyethylene glycol derivatives of stearic acid, for example, PEG-30 stearate, PEG-40 stearate, PEG-50 stearate, PEG-75 stearate, PEG-90 stearate, PEG-100 stearate, PEG-120 stearate, and PEG-150 stearate. Preferred for use in the compositions herein is PEG-100 stearate.

Polyoxyethylene/polyoxypropylene copolymers can include, for example, polyoxyethylene/polyoxypropylene random copolymer and polyoxyethylene/polyoxypropylene block copolymer. Among these polyoxyalkylene derivatives, polyoxyethylene/polyoxypropylene copolymers including polyoxyethylene/polyoxypropylene random copolymer and polyoxyethylene/polyoxypropylene block copolymer can be used in the composition as described herein in view of their suspending benefit. Polyoxyethylene/polyoxypropylene block copolymer can be used and in some examples the composition can include polyoxyethylene/polyoxypropylene block copolymer having a weight ratio of polyoxyethylene to polyoxypropylene of from 5:10 to 8:10, alternatively the block copolymer having the ratio of 8:10.

Commercially available polyoxyalkylene derivatives useful herein include: polyoxyethylene/polyoxypropylene block copolymer; having CTFA name Poloxamer 338, available from BASF^{®} under the name Pluronic^{®} F-108, and also available from Sanyo^{®} Chemical under the name Newpol^{®} PE-108; and having CTFA name Poloxamer 288, available from BASF^{®} under the name Pluronic^{®} F-98, and also available from Sanyo^{®} Chemical under the name Newpol^{®} PE-98.

### Inert Carrier

The anhydrous first composition as described herein preferably comprises an inert carrier. The inert carrier can be included in the first composition at a level by weight of from 3% to 90%, alternatively from 25% to 90%, alternatively from 30% to 85%, and alternatively from 10% to 70%, by weight of the first composition.

The inert carriers useful herein can include liquid carriers, for example, liquid polyhydric alcohols such as polyethylene glycol, polypropylene glycol, 1,2-propane diol or propylene glycol, 1,3-propane diol, hexylene glycol, glycerin, diethylene glycol, dipropylene glycol, 1,2-butylene glycol, 1,4-butylene glycol, ethanol, sorbitol, diglycerin, polyglycerols; liquid paraffin; mineral oil; vegetable oil; low melting point oil such as pentaerythritol tetraisostearate; and mixtures thereof. The liquid polyhydric alcohols such as polyethylene glycol can also be used as additional heat generating agents. Preferred are polyethylene glycol, polypropylene glycol, glycerin, diglycerin, sorbitol, liquid paraffin, mineral oil, vegetable oil, pentaerythritol tetraisostearate, and mixtures thereof in view of physical properties such as viscosity and fluidity. More preferred is polyethylene glycol in view of its ability to generate a heat by mixing with water and physical properties such as viscosity and fluidity.

The polyethylene glycols useful herein are those having the formula:

H(OCH₂CH₂)n-OH

wherein n has an average value of from 4 to 12.

The polyethylene glycol described above is also known as a polyethylene oxide, and polyoxyethylene. Polyethylene glycols useful herein that are especially preferred are PEG-200 wherein n has an average value of 4. Commercially available preferred polyethylene glycol
includes, for example, PEG-4 having trade name Pluracare E 200 available from BASF.

### Reaction Control Agents

The anhydrous first compositions as described herein preferably contain reaction control agents which can control the heat generating reaction of the inorganic heat generating agent. The reaction control agents may slow down the reaction or accelerate the reaction. The reaction control agents may also control the temperature to which the first composition warms up.

Acids can be used as reaction control agents for accelerating the reaction of the inorganic heat generating agents. The acid useful herein includes, for example, citric acid, sodium diphosphate, potassium diphosphate, l-glutamic acid, lactic acid, hydrochloric acid, malic acid, succinic acid, acetic acid, fumaric acid, l-glutamic acid hydrochloride, tartaric acid, and mixtures thereof; preferably l-glutamic acid, lactic acid, hydrochloric acid, and mixtures thereof. Among the above acids, citric acid is preferably used herein. Some acids can also be used together with amidoamines for providing conditioning benefits as described below. The acid can be contained at a level such that the mole ratio of the inorganic heat generating agent to acid is from 1:0.1 to 1:10, preferably from 1:0.5 to 1:5. Water absorbing polymer can be used as reaction control agents for slowing down the reaction of the inorganic heat generating agent. The water absorbing polymer useful herein includes, for example, vinyl polymers such as crosslinked acrylic acid polymers with the CTFA name Carbomer, carboxylic acid/carboxylate copolymers such as acrylic acid/alkyl acrylate copolymers with the CTFA name Acrylates/C10-30 Alkyl Acrylate Crosspolymer, cellulose derivatives and modified cellulose polymers such as hydroxyethylcellulose and hydroxypropyl cellulose, polyvinylpyrrolidone, polyvinyl alcohol, guar gum, other gums, starch-based polymers, alginic acid-based polymers, acrylate polymers, polyalkylene glycols having a molecular weight of more than 1000, and mixtures thereof. These water absorbing polymers can also be used as viscosity modifying agents, described below.

Among the above water absorbing polymers, preferred are cellulose derivatives and modified cellulose polymers, and more preferred is Hydroxyethylcellulose. The water absorbing polymers can be included in the first composition, at a level by weight of, preferably from 0.2% to 20%, more preferably from 0.5% to 15%, still more preferably from 1% to 10%.

### Heat Reserving Materials

The anhydrous first compositions as described herein may contain heat reserving materials which can reserve a heat. The heat reserving material can be used for prolonging heating, and may be used for slowing down the warming speed, and may also control the temperature to which the cosmetic composition warms up.

The heat reserving materials include, for example, silica gel, carboxymethyl cellulose gel, phase-changing materials, and mixtures thereof. The phase-changing materials useful herein are those which have a melting point of from 25°C to 80°C. The phase-changing materials useful herein include, for example, a fatty compound such as fatty alcohol and fatty acid; hydrocarbons; a mixture of hydrocarbons and foamed polyolefin; and mixtures thereof. Fatty compound useful herein are disclosed below as high melting point fatty compounds.

The heat reserving material can be included in the first compositions at a level by weight of, preferably from 0.2% to 20%, more preferably from 0.5% to 15% still more preferably from 1% to 10%.

### Viscosity Modifying Agent

The anhydrous first composition as described herein may contain a viscosity modifying agent. The viscosity modifying agent useful herein includes, for example, vinyl polymers such as cross linked acrylic acid polymers with the CTFA name Carbomer, carboxylic acid/carboxylate copolymers such as acrylic acid/alkyl acrylate copolymers with the CTFA name Acrylates/C10-30 Alkyl Acrylate Crosspolymer, cellulose derivatives and modified cellulose polymers, polyvinylpyrrolidone, polyvinyl alcohol, guar gum, other gums, starch-based polymers, alginic acid-based polymers, acrylate polymers, polyalkylene glycols having a molecular weight of more than 1000, inorganic water soluble material such as bentonite, aluminum magnesium silicate, laponite, hectorite, and anhydrous silicic acid, and mixtures thereof. The polymers described herein can also be used as the viscosity modifying agent. Some polyalkylene glycols described herein can also be used as hair conditioning agents.

The viscosity modifying agent can be included in the compositions at a level by weight of, preferably from 0.01% to 5%, more preferably from 0.05% to 3% still more preferably from 0.1% to 3%.

### Additional Heat Generating Agents

The anhydrous first compositions as described herein may contain additional heat generating agents, in addition to the inorganic heat generating agents, which generate a heat by mixing with water. Such additional heat generating agents useful herein include, for example, organic heat generating agents such as polyhydric alcohols.

The polyhydric alcohol useful herein includes, for example, polyethylene glycol, polypropylene glycol, 1,2-propane diol or propylene glycol, 1,3-propane diol, hexylene glycol, glycerin, diethylene glycol, dipropylene glycol, 1,2-butylene glycol, 1,4-butylene glycol, ethanol, **1-**propanol, 1-butanol, 2-propanol, erythritol, threitol, xylitol, sorbitol, mannitol, galactitol, iditol, inositol, diglycerin, polyglycerols, polyglycerol fatty acid esters, and mixtures thereof. These can also be used as the inert carrier described above.

Such additional heat generating agents can be included in the first compositions at a level by weight of, alternatively from 2% to 85%, alternatively from 5% to 85%, and alternatively from 10% to 85%.

### Hair Conditioning Composition

The anhydrous composition can include can a hair conditioning composition. The anhydrous hair compositions can contain hair conditioning agents in addition to the above-described heat generating agent, the phase changing agent, and the inert carrier. The hair conditioning agents useful herein include, for example, high melting point fatty compounds, amidoamines, acids, cationic conditioning agents such as cationic surfactants and cationic polymers, low melting point oils, silicone compounds, polypropylene glycol, polyethylene glycol, and mixtures thereof.

Among these hair conditioning agents, preferred are high melting point fatty compounds, amidoamines, acids and mixtures thereof.

### High Melting Point Fatty Compound

The hair conditioning composition as described herein preferably comprises a high melting point fatty compound. The high melting point fatty compound can be used as the "Phase Changing Agent" described above.

The high melting point fatty compound useful herein have a melting point of 25°C or higher, and is selected from the group consisting of fatty alcohols, fatty acids, fatty alcohol derivatives, fatty acid derivatives, and mixtures thereof. It is understood by the artisan that the compounds disclosed in this section of the specification can in some instances fall into more than one classification, e.g., some fatty alcohol derivatives can also be classified as fatty acid derivatives. However, a given classification is not intended to be a limitation on that particular compound, but is done so for convenience of classification and nomenclature. Further, it is understood by the artisan that, depending on the number and position of double bonds, and length and position of the branches, certain compounds having certain required carbon atoms may have a melting point of less than 25°C. Such compounds of low melting point are not intended to be included in this section. Nonlimiting examples of the high melting point compounds are found in International Cosmetic Ingredient Dictionary, Fifth Edition, 1993, and CTFA Cosmetic Ingredient Handbook, Second Edition, 1992.

The high melting point fatty compound can be included in the composition at a level by weight of, preferably from 0.1% to 30%, more preferably from 0.2% to 0.25%, still more preferably from 0.5% to 15%.

The fatty alcohols useful herein are those having from 14 to 30 carbon atoms, alternatively from 16 to 22 carbon atoms. These fatty alcohols are saturated and can be straight or branched chain alcohols. Nonlimiting examples of fatty alcohols include, cetyl alcohol, stearyl alcohol, behenyl alcohol, and mixtures thereof.

The fatty acids useful herein are those having from 10 to 30 carbon atoms, alternatively from 12 to 22 carbon atoms, and alternatively from 16 to 22 carbon atoms. These fatty acids are saturated and can be straight or branched chain acids. Also included are diacids, triacids, and other multiple acids which meet the requirements herein. Also included herein are salts of these fatty acids. Nonlimiting examples of fatty acids can include lauric acid, palmitic acid, stearic acid, behenic acid, sebacic acid, and mixtures thereof.

The fatty alcohol derivatives and fatty acid derivatives useful herein include alkyl ethers of fatty alcohols, alkoxylated fatty alcohols, alkyl ethers of alkoxylated fatty alcohols, esters of fatty alcohols, fatty acid esters of compounds having esterifiable hydroxy groups, hydroxy-substituted fatty acids, and mixtures thereof. Nonlimiting examples of fatty alcohol derivatives and fatty acid derivatives include materials such as methyl stearyl ether; the ceteth series of compounds such as ceteth-1 through ceteth-45, which are ethylene glycol ethers of cetyl alcohol, wherein the numeric designation indicates the number of ethylene glycol moieties present; the steareth series of compounds such as steareth-1 through 10, which are ethylene glycol ethers of steareth alcohol, wherein the numeric designation indicates the number of ethylene glycol moieties present; ceteareth 1 through ceteareth-10, which are the ethylene glycol ethers of ceteareth alcohol, i.e. a mixture of fatty alcohols containing predominantly cetyl and stearyl alcohol, wherein the numeric designation indicates the number of ethylene glycol moieties present; C 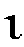 -C₃₀ alkyl ethers of the ceteth, steareth, and ceteareth compounds just described; polyoxyethylene ethers of behenyl alcohol; ethyl stearate, cetyl stearate, cetyl palmitate, stearyl stearate, myristyl myristate, polyoxyethylene cetyl ether stearate, polyoxyethylene stearyl ether stearate, polyoxyethylene lauryl ether stearate, ethyleneglycol monostearate, polyoxyethylene monostearate, polyoxyethylene distearate, propyleneglycol monostearate, propyleneglycol distearate, trimethylolpropane distearate, sorbitan stearate, polyglyceryl stearate, glyceryl monostearate, glyceryl distearate, glyceryl tristearate, and mixtures thereof.

High melting point fatty compounds of a single compound of high purity are preferred. Single compounds of pure fatty alcohols selected from the group of pure cetyl alcohol, stearyl alcohol, and behenyl alcohol can be preferred. By "pure" herein, what is meant is that the compound has a purity of at least 90%, preferably at least 95%. These single compounds of high purity provide good rinsability from the hair when the consumer rinses off the composition.

Commercially available high melting point fatty compounds useful herein include: cetyl alcohol, stearyl alcohol, and behenyl alcohol having tradenames CONOL series available from Shin Nihon Rika (Osaka, Japan), and NAA series available from NOF (Tokyo, Japan).

### Amidoamine

The hair conditioning composition as described herein preferably comprises an amidoamine of the following general formula:

R¹ CONH (CH₂)ₘ N (R²)₂

wherein R¹ is a residue of C₁₁ to C₂₄ fatty acids, R² is a C₁ to C₄ alkyl, and m is an integer from 1 to 4.

The amidoamine can be included in the composition at a level by weight of, preferably from 0.05% to 10%, more preferably from 0.05% to 8%, still more preferably from 0.1% to 5%.

Amidoamines useful in can include stearamidopropyldimethylamine, stearamidopropyldiethylamine, stearamidoethyldiethylamine, stearamidoethyldimethylamine, palmitamidopropyldimethylamine, palmitamidopropyldiethylamine, palmitamidoethyldiethylamine, palmitamidoethyldimethylamine, behenamidopropyldimethylamine, behenamidopropyldiethylamine, behenamidoethyldiethylamine, behenamidoethyldimethylamine, arachidamidopropyldimethylamine, arachidamidopropyldiethylamine, arachidamidoethyldiethylamine, arachidamidoethyldimethylamme, and mixtures thereof; more preferably stearamidopropyldimethylamine, stearamidoethyldiethylamine, and mixtures thereof. Commercially available amidoamines useful herein include: stearamidopropyldimethylamine having tradename SAPDMA available from Inolex, and tradename Amidoamine MPS available from Nikko.

### Acids

The hair conditioning composition as described herein preferably comprises an acid selected from the group consisting of L-glutamic acid, lactic acid, hydrochloric acid, malic acid, succinic acid, acetic acid, fumaric acid, L-glutamic acid hydrochloride, tartaric acid, and mixtures thereof; preferably L-glutamic acid, lactic acid, hydrochloric acid, and mixtures thereof. The acid described herein can also be used as the "Reaction Control Agent" described above. The acid can be contained at a level such that the mole ratio of amidoamine to acid is, preferably from 1:0.3 to 1:1, more preferably from 1:0.5 to 1:0.9.

Commercially available acids useful herein include: L-Glutamic acid; L- Glutamic acid (cosmetic grade) available from Ajinomoto.

### Cationic Conditioning Agent

The hair conditioning composition as described herein may contain a cationic conditioning agent.

The cationic conditioning agent can be included in the composition at a level by weight of, preferably from 0.1 % to 10%, alternatively from 0.25% to 8%, alternatively from 0.5% to 3%.

The cationic conditioning agent is selected from the group consisting of cationic surfactants, cationic polymers, and mixtures thereof.

### Cationic surfactant

The cationic surfactant useful herein is any known to the artisan and described below.

Among the cationic surfactants useful herein are those corresponding to the general formula (I): 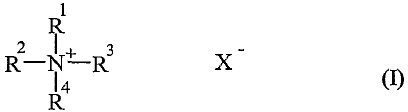 wherein at least one of R¹, R², R³, and R⁴ is selected from an aliphatic group of from 8 to 30 carbon atoms or an aromatic, alkoxy, polyoxyalkylene, alkylamido, hydroxyalkyl, aryl or alkylaryl group having up to 22 carbon atoms, the remainder of R¹, R², R³, and R⁴ are independently selected from an aliphatic group of from 1 to 22 carbon atoms or an aromatic, alkoxy, polyoxyalkylene, alkylamido, hydroxyalkyl, aryl or alkylaryl group having up to 22 carbon atoms; and X is a salt-forming anion such as those selected from halogen, (e.g. chloride, bromide), acetate, citrate, lactate, glycolate, phosphate, nitrate, sulfonate, sulfate, alkylsulfate, and alkyl sulfonate radicals. The aliphatic groups can contain, in addition to carbon and hydrogen atoms, ether linkages, and other groups such as amino groups. The longer chain aliphatic groups, e.g., those of 12 carbons, or higher, can be saturated or unsaturated. Preferred is when R¹, R², R³, and R⁴ are independently selected from Ci to C₂₂ alkyl. Nonlimiting examples of cationic surfactants useful herein include the materials having the following CTFA designations: quaternium-8, quaternium-14, quaternium-18, quaternium-18 methosulfate, quaternium-24, and mixtures thereof.

Among the cationic surfactants of general formula (I), preferred are those containing in the molecule at least one alkyl chain having at least 16 carbons. Nonlimiting examples of such preferred cationic surfactants include: behenyl trimethyl ammonium chloride available, for example, with tradename INCROQUAT TMC-80 from Croda and ECONOL TM22 from Sanyo Kasei; cetyl trimethyl ammonium chloride available, for example, with tradename CA-2350 from Nikko Chemicals, hydrogenated tallow alkyl trimethyl ammonium chloride, dialkyl (14-18) dimethyl ammonium chloride, ditallow alkyl dimethyl ammonium chloride, dihydrogenated tallow alkyl dimethyl ammonium chloride, distearyl dimethyl ammonium chloride, dicetyl dimethyl ammonium chloride, di(behenyl/arachidyl) dimethyl ammonium chloride, dibehenyl dimethyl ammonium chloride, stearyl dimethyl benzyl ammonium chloride, stearyl propyleneglycol phosphate dimethyl ammonium chloride, stearoyl amidopropyl dimethyl benzyl ammonium chloride, stearoyl amidopropyl dimethyl (myristylacetate) ammonium chloride, and N-(stearoyI colamino formyl methyl) pyridinium chloride.

Also preferred are hydrophilically substituted cationic surfactants in which at least one of the substituents contain one or more aromatic, ether, ester, amido, or amino moieties present as substituents or as linkages in the radical chain, wherein at least one of the R¹ - R⁴ radicals contain one or more hydrophilic moieties selected from alkoxy (preferably C₁ - C₃ alkoxy), polyoxyalkylene (preferably C₁ - C₃ polyoxyalkylene), alkylamido, hydroxyalkyl, alkylester, and combinations thereof. Preferably, the hydrophilically substituted cationic conditioning surfactant contains from 2 to 10 nonionic hydrophile moieties located within the above stated ranges. Preferred hydrophilically substituted cationic surfactants include those of the formula (II) through (VIII) below: 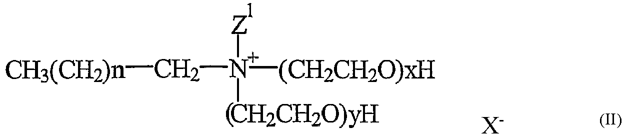 wherein n is from 8 to 28, x+y is from 2 to 40, Z¹ is a short chain alkyl, preferably a C₁ - C₃ alkyl, more preferably methyl, or (CH₂CH₂O)zH wherein x+y+z is up to 60, and X is a salt forming anion as defined above; 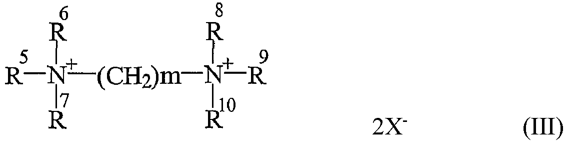 wherein m is 1 to 5, one or more of R⁵, R⁶, and R⁷ are independently an C₁ - C₃₀ alkyl, the remainder are CH₂CH₂OH, one or two of R⁸, R⁹, and R¹⁰ are independently an C₁ - C₃₀ alkyl, and remainder are CH₂CH₂OH, and X is a salt forming anion as mentioned above; 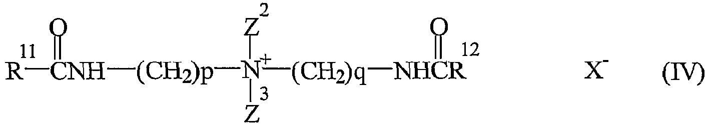 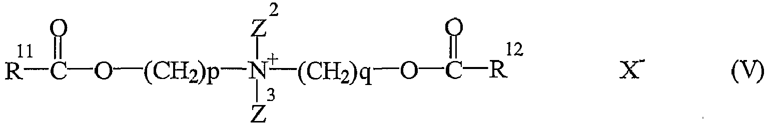 wherein, independently for formulae (IV) and (V), Z² is an alkyl, preferably a C₁ - C₃ alkyl, more preferably methyl, and Z³ is a short chain hydroxyalkyl, preferably hydroxymethyl or hydroxyethyl, p and q independently are integers from 2 to 4, inclusive, preferably from 2 to 3, inclusive, more preferably 2, R¹¹ and R¹² , independently, are substituted or unsubstituted hydrocarbyls, preferably C₁₂ - C₂₀ alkyl or alkenyl, and X is a salt forming anion as defined above; 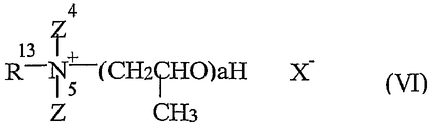 wherein R¹³ is a hydrocarbyl, preferably a C₁ - C₃ alkyl, more preferably methyl, Z⁴ and Z⁵ are, independently, short chain hydrocarbyls, preferably C₂ - C₄ alkyl or alkenyl, more preferably ethyl, a is from 2 to 40, preferably from 7 to 30, and X is a salt forming anion as defined above; 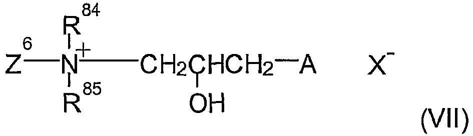 wherein R⁸⁴ and R⁸⁵, independently, are C₁ - C₃ alkyl, preferably methyl, Z⁶ is a C₁₂ - C₂₂ hydrocarbyl, alkyl carboxy or alkylamido, and A is a protein, preferably a collagen, keratin, milk protein, silk, soy protein, wheat protein, or hydrolyzed forms thereof; and X is a salt forming anion as defined above; 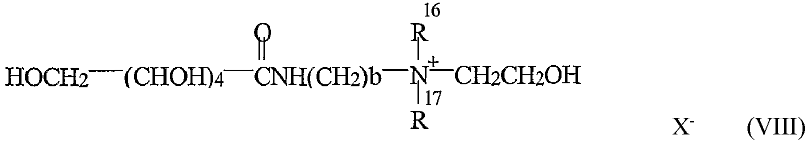 wherein b is 2 or 3, R¹⁶ and R¹⁷, independently are C₁ - C₃ hydrocarbyls preferably methyl, and X is a salt forming anion as defined above. Nonlimiting examples of hydrophilically substituted cationic surfactants useful herein include the materials having the following CTFA designations: quaternium-16, quaternium-26, quaternium-27, quaternium-30, quaternium-33, quaternium-43, quatemium-52, quatemium-53, quatemium-56, quaternium-60, quaternium-61 , quaternium-62, quatemium-70, quatemium-71 , quaternium-72, quatemium-75, quatemium-76 hydrolyzed collagen, quatemium-77, quatemium- 78, quaternium-79 hydrolyzed collagen, quaternium-79 hydrolyzed keratin, quaternium-79 hydrolyzed milk protein, quaternium-79 hydrolyzed silk, quaternium-79 hydrolyzed soy protein, and quaternium-79 hydrolyzed wheat protein, quatemium-80, quatemium-81 , quatemium-82, quaternium-83, quaternium-84, and mixtures thereof.

Highly preferred hydrophilically substituted cationic surfactants include dialkylamido ethyl hydroxyethylmonium salt, dialkylamidoethyl dimonium salt, dialkyloyl ethyl hydroxyethylmonium salt, dialkyloyl ethyldimonium salt, and mixtures thereof; for example, commercially available under the following tradename; TETRANYL CO-40 from Kao Chemicals.

### Second Composition (Conditioning Composition)

The second composition can comprise an oil phase and an aqueous phase.

### Details of the Oil Phase Composition

The oil phase may comprise the surfactants and the high melting point fatty compounds. The oil phase comprises preferably from 50% to 100%, more preferably from 60% to 100%, still more preferably from 70% to 100% of the surfactants and the high melting point fatty compounds, by weight of the total amount of the surfactants and the high melting point fatty compounds used in the second composition.

The surfactants and the high melting point fatty compounds are present in the oil phase, with or without other ingredients, at a level by weight of the oil phase of, preferably from 35% to 100%, more preferably from 50% to 100%, still more preferably from 60% to 100%.

Oil phase may contain an aqueous carrier such as water and lower alkyl alcohols, and polyhydric alcohols. If included, the level of aqueous carrier in the oil phase is up to 50%, more preferably up to 40%, still more preferably up to 25%, even more preferably up to 15% by weight of the oil phase, in view of providing the benefits. Among the aqueous carrier, it is further preferred to control the level of water in oil phase, such that the level of water in oil phase is preferably up to 40%, more preferably up to 25%, still more preferably up to 15%, even more preferably up to 10% by weight of the oil phase. The oil phase may be substantially free of water. As used herein, "oil phase being substantially free of water" means that: the oil phase is free of water; the oil phase contains no water other than impurities of the ingredients; or, if the oil phase contains water, the level of such water is very low. A total level of such water in the oil phase, if included, may be preferably 1% or less, more preferably 0.5% or less, still more preferably 0.1% or less by weight of the oil phase.

The oil phase may contain other ingredients than the surfactants and the high melting point fatty compounds and aqueous carrier. Such other ingredients are, for example, water-insoluble components and/or heat sensitive components, such as water-insoluble silicones, water-insoluble perfumes, water-insoluble preservatives such as parabens and non-heat sensitive preservatives such as benzyl alcohol. As used herein, "water-insoluble components" means that the components have a solubility in water at 25°C of below 1g/100g water (excluding 1g/100 water), preferably 0.7g/100g water or less, more preferably 0.5g/100g water or less, still more preferably 0.3g/100g water or less. If included, it is preferred that the level of such other ingredients in the oil phase is up to 50%, more preferably up to 40%, by weight of the oil phase, in view of providing the benefits.

### Details of the Aqueous Phase Composition

The aqueous phase comprises an aqueous carrier. The aqueous phase comprises preferably from 50% to 100%, more preferably from 70% to 100%, still more preferably from 90% to 100%, even more preferably from 95% to 100% of aqueous carrier, by weight of the total amount of the aqueous carrier used in the second composition.

The aqueous carrier is present in the aqueous phase, with or without other ingredients, at a level by weight of the aqueous phase of, from 50% to 100%, more preferably from 70% to 100%, still more preferably from 90% to 100%, even more preferably from 95% to 100%.

The aqueous phase may contain the surfactants and high melting point fatty compounds. If included, it is preferred that the level of the sum of the surfactants and high melting point fatty compounds in the aqueous phase is up to 20%, more preferably up to 10%, still more preferably up to 7% by weight of the aqueous phase, in view of providing the benefits. Even more preferably, the aqueous phase is substantially free of the surfactants and high melting point fatty compounds. As used herein, "aqueous phase being substantially free of the surfactants and high melting point fatty compounds" means that: the aqueous phase is free of the surfactants and high melting point fatty compounds; or, if the aqueous phase contains the surfactants and high melting point fatty compounds, the level of such surfactants and high melting point fatty compounds is very low. A total level of such surfactants and high melting point fatty compounds in the aqueous phase, if included, may be preferably 1% or less, more preferably 0.5% or less, still more preferably 0.1% or less by weight of the aqueous phase.

The aqueous phase may contain other ingredients than the surfactants and the high melting point fatty compounds and aqueous carrier. Such other ingredients are, for example, water soluble components and/or heat sensitive components, such as water soluble pH adjusters, water soluble polymers and water soluble preservatives such as phenoxyethanol, Kathon^{®} and sodium benzoate. As used herein, "water soluble components" means that the components have a solubility in water at 25°C of at least 1g/100g water, preferably at least 1.2g/100g water, more preferably at least 1.5g/100g water, still more preferably at least 2.0g/100 water. If included, it is preferred that the level of such other ingredients in the aqueous phase is up to 20%, more preferably up to 10% by weight of the aqueous phase, in view of providing the benefits.

The second composition as described herein may comprise a surfactant, high melting point fatty compound, and aqueous carrier. The surfactants, the high melting point fatty compounds, and the aqueous carrier are in the form of emulsion.

### Cationic Surfactant System

The compositions as described herein may comprise a cationic surfactant system. The cationic surfactant system can be included in the composition at a level from 0.5%,_preferably from 1%, more preferably from 1.5%, still more preferably from 1.8%, still more preferably from 2.0%, and to 8%, preferably to 5%, more preferably to 4% by weight of the second composition, in view of providing the benefits.

Preferably, the surfactant may be water-insoluble. as used herein, "water-insoluble surfactants" means that the surfactants have a solubility in water at 25°C of below 1g/100g water (excluding 1g/100 water), preferably 0.7g/100g water or less, more preferably 0.5g/100g water or less, still more preferably 0.3g/100g water or less.

Cationic surfactant system useful herein comprises a mono-alkyl quaternized ammonium salt cationic surfactant and some aspects may include a di-alkyl cationic surfactant. It is believed that such combination of a mono-alkyl quaternized ammonium salt cationic surfactant and a di-alkyl cationic surfactant provides feel of quick rinse and/or feel of easy to spread through hair, compared to single use of mono-alkyl cationic surfactants which have one long alkyl chain which has from 12 to 30 carbon atoms. In the cationic surfactant system it is preferred that the weight ratio of the mono-alkyl quaternized ammonium salt cationic surfactant to the di-alkyl cationic surfactant is from 1:1 to 10:1, more preferably from 1.5:1 to 7:1, still more preferably from 2:1 to 5:1, in view of stability in rheology and conditioning benefits.

### Mono-alkyl quaternized ammonium salt cationic surfactant

The mono-alkyl quaternized ammonium salt cationic surfactants useful herein are those having one long alkyl chain of preferably from 12 to 30 carbon atoms, more preferably from 16 to 24 carbon atoms, still more preferably from 18 to 22 carbon atoms, in view of conditioning benefits. Such mono-alkyl quaternized ammonium salt cationic surfactants useful herein are, for example, those having the formula (I): 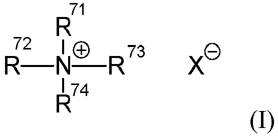 wherein one of R⁷¹, R⁷², R⁷³ and R⁷⁴ is selected from an aliphatic group of from 12 to 30 carbon atoms, more preferably from 16 to 24 carbon atoms, still more preferably from 18 to 22 carbon atoms or an aromatic, alkoxy, polyoxyalkylene, alkylamido, hydroxyalkyl, aryl or alkylaryl group having up to 30 carbon atoms; the remainder of R⁷¹, R⁷², R⁷³ and R⁷⁴ are independently selected from an aliphatic group of from 1 to 8 carbon atoms, preferably from 1 to 3 carbon atoms or an aromatic, alkoxy, polyoxyalkylene, alkylamido, hydroxyalkyl, aryl or alkylaryl group having up to 8 carbon atoms; and X⁻ is a salt-forming anion selected from the group consisting of halides such as chloride and bromide, C1-C4 alkyl sulfate such as methosulfate and ethosulfate, and mixtures thereof. The aliphatic groups can contain, in addition to carbon and hydrogen atoms, ether linkages, and other groups such as amino groups. The longer chain aliphatic groups, *e.g.,* those of 16 carbons, or higher, can be saturated or unsaturated. Preferably, one of R⁷¹, R⁷², R⁷³ and R⁷⁴ is selected from an alkyl group of from 12 to 30 carbon atoms, more preferably from 16 to 24 carbon atoms, still more preferably from 18 to 22 carbon atoms; and the remainder of R⁷¹, R⁷², R⁷³ and R⁷⁴ are independently selected from CH₃, C₂H₅, C₂H₄OH, CH₂C₆H₅, and mixtures thereof.

Among them, more preferred cationic surfactants are those having a longer alkyl group, i.e., C18-22 alkyl group. Such cationic surfactants include, for example, behenyl trimethyl ammonium chloride, methyl sulfate or ethyl sulfate, and stearyl trimethyl ammonium chloride, methyl sulfate or ethyl sulfate.

### Di-alkyl cationic surfactant

Di-alkyl cationic surfactants useful herein are those having two long alkyl chains of from 12 to 30 carbon atoms, more preferably from 16 to 24 carbon atoms, still more preferably from 18 to 22 carbon atoms, including, for example, di-long alkyl quaternized ammonium salts. Such di-alkyl quaternized ammonium salts useful herein are those having the formula (I): 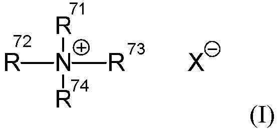 wherein two of R⁷¹, R⁷², R⁷³ and R⁷⁴ are selected from an aliphatic group of from 12 to 30 carbon atoms, preferably from 16 to 24 carbon atoms, more preferably from 18 to 22 carbon atoms or an aromatic, alkoxy, polyoxyalkylene, alkylamido, hydroxyalkyl, aryl or alkylaryl group having up to 30 carbon atoms; the remainder of R⁷¹, R⁷², R⁷³ and R⁷⁴ are independently selected from an aliphatic group of from 1 to 8 carbon atoms, preferably from 1 to 3 carbon atoms or an aromatic, alkoxy, polyoxyalkylene, alkylamido, hydroxyalkyl, aryl or alkylaryl group having up to 8 carbon atoms; and X⁻ is a salt-forming anion selected from the group consisting of halides such as chloride and bromide, C1-C4 alkyl sulfate such as methosulfate and ethosulfate, and mixtures thereof. The aliphatic groups can contain, in addition to carbon and hydrogen atoms, ether linkages, and other groups such as amino groups. The longer chain aliphatic groups, e.g., those of 16 carbons, or higher, can be saturated or unsaturated. Preferably, two of R⁷¹, R⁷², R⁷³ and R⁷⁴ are selected from an alkyl group of from 12 to 30 carbon atoms, preferably from 16 to 24 carbon atoms, more preferably from 18 to 22 carbon atoms; and the remainder of R⁷¹, R⁷², R⁷³ and R⁷⁴ are independently selected from CH₃, C₂H₅, C₂H₄OH, CH₂C₆H₅, and mixtures thereof.

Such preferred di-alkyl cationic surfactants include, for example, dialkyl (14-18) dimethyl ammonium chloride, ditallow alkyl dimethyl ammonium chloride, dihydrogenated tallow alkyl dimethyl ammonium chloride, distearyl dimethyl ammonium chloride, and dicetyl dimethyl ammonium chloride.

### High Melting Point Fatty Compound

The high melting point fatty compound can be included in the second composition at a level of from 0.5%, preferably from 1.0%, more preferably form 1.5%, still more preferably from 2%, even more preferably from 4%, and to 15%, preferably to 6% by weight of the second composition, in view of providing the benefits.

The high melting point fatty compound useful herein have a melting point of 25°C or higher, preferably 40°C or higher, more preferably 45°C or higher, still more preferably 50°C or higher, in view of stability of the emulsion, especially the gel matrix. Preferably, such melting point is up to 90°C, more preferably up to 80°C, still more preferably up to 70°C, even more preferably up to 65°C, in view of easier manufacturing and easier emulsification. The high melting point fatty compound can be used as a single compound or as a blend or mixture of at least two high melting point fatty compounds. When used as such blend or mixture, the above melting point means the melting point of the blend or mixture.

The high melting point fatty compound useful herein is selected from the group consisting of fatty alcohols, fatty acids, fatty alcohol derivatives, fatty acid derivatives, and mixtures thereof. It is understood by the artisan that the compounds disclosed in this section of the specification can in some instances fall into more than one classification, e.g., some fatty alcohol derivatives can also be classified as fatty acid derivatives. However, a given classification is not intended to be a limitation on that particular compound, but is done so for convenience of classification and nomenclature. Further, it is understood by the artisan that, depending on the number and position of double bonds, and length and position of the branches, certain compounds having certain required carbon atoms may have a melting point of less than the above preferred. Such compounds of low melting point are not intended to be included in this section. Nonlimiting examples of the high melting point compounds are found in International Cosmetic Ingredient Dictionary, Fifth Edition, 1993, and CTFA Cosmetic Ingredient Handbook, Second Edition, 1992.

Among a variety of high melting point fatty compounds, fatty alcohols may be used in the composition as described herein. The fatty alcohols useful herein are those having from 14 to 30 carbon atoms, preferably from 16 to 22 carbon atoms. These fatty alcohols are saturated and can be straight or branched chain alcohols.

Preferred fatty alcohols include, for example, cetyl alcohol (having a melting point of 56°C), stearyl alcohol (having a melting point of 58-59°C), behenyl alcohol (having a melting point of 71°C), and mixtures thereof. These compounds are known to have the above melting point. However, they often have lower melting points when supplied, since such supplied products are often mixtures of fatty alcohols having alkyl chain length distribution in which the main alkyl chain is cetyl, stearyl or behenyl group. More preferred fatty alcohols may be cetyl alcohol, stearyl alcohol and mixtures thereof.

Commercially available high melting point fatty compounds useful herein include: cetyl alcohol, stearyl alcohol, and behenyl alcohol having tradenames CONOL series available from Shin Nihon Rika (Osaka, Japan), and NAA series available from NOF (Tokyo, Japan).

### Gel Matrix

Preferably, the emulsion of the second composition is in the form of a gel matrix. The gel matrix comprises the cationic surfactant system, the high melting point fatty compound, and an aqueous carrier. The gel matrix is suitable for providing various conditioning benefits, such as a slippery feel during the application to wet hair and softness and moisturized feel on dry hair.

Preferably, especially when the gel matrix is formed, the total amount of the cationic surfactant and the high melting point fatty compound is from 1.0%, preferably from 2.0%, more preferably from 3.0% by weight of the composition, in view of providing the benefits, and to 15%, preferably to 14%, more preferably to 13%, still more preferably to 10% by weight of the composition, in view of spreadability and product appearance. Furthermore, when the gel matrix is formed, the cationic surfactant and the high melting point fatty compound are contained at a level such that the weight ratio of the cationic surfactant to the high melting point fatty compound is in the range of, preferably from 1:1 to 1:10, more preferably from 1:1 to 1:4, still more preferably from 1:2 to 1:4, in view of providing improved wet conditioning benefits.

Preferably, when the gel matrix is formed, the composition as described herein is substantially free of anionic surfactants and anionic polymers, in view of stability of the gel matrix. As used herein, "the composition being substantially free of anionic surfactants and anionic polymers" means that the composition is free of anionic surfactants and anionic polymers; or, if the composition contains anionic surfactants and anionic polymers, the level of such anionic surfactants and anionic polymers is very low. A total level of such anionic surfactants and anionic polymers, if included, may be preferably 1% or less, more preferably 0.5% or less, still more preferably 0.1% or less by weight of the composition. Most preferably, the total level of such anionic surfactants and anionic polymers is 0% by weight of the composition.

### Aqueous Carrier

The second composition as described herein may comprise an aqueous carrier. The level and species of the carrier are selected according to the compatibility with other components and other desired characteristic of the product.

The carrier useful herein includes water and water solutions of lower alkyl alcohols and polyhydric alcohols. The lower alkyl alcohols useful herein are monohydric alcohols having 1 to 6 carbons, more preferably ethanol and isopropanol. The polyhydric alcohols useful herein include propylene glycol, hexylene glycol, glycerin, and propane diol.

Preferably, the aqueous carrier is substantially water. Deionized water is preferably used. Water from natural sources including mineral cations can also be used, depending on the desired characteristic of the product. Generally, the second compositions as described herein comprise from 20% to 99%, preferably from 30% to 95%, and more preferably from 80% to 90% watery, by weight of the second composition.

### Silicone Compound

The second composition may contain a silicone compound. It is believed that the silicone compound can provide smoothness and softness on dry hair. The silicone compounds herein can be used at levels by weight of the second composition of preferably from 0.1% to 20%, more preferably from 0.5% to 10%, still more preferably from 1% to 8%.

Preferably, the silicone compounds may have an average particle size of from 1microns to 50 microns, in the composition.

The silicone compounds useful herein, as a single compound, as a blend or mixture of at least two silicone compounds, or as a blend or mixture of at least one silicone compound and at least one solvent, have a viscosity of preferably from 1,000 to 2,000,000mPa·s at 25°C.

The viscosity can be measured by means of a glass capillary viscometer as set forth in Dow Corning Corporate Test Method CTM0004, July 20, 1970. Suitable silicone fluids include polyalkyl siloxanes, polyaryl siloxanes, polyalkylaryl siloxanes, polyether siloxane copolymers, amino substituted silicones, quaternized silicones, and mixtures thereof. Other nonvolatile silicone compounds having conditioning properties can also be used.

Preferred polyalkyl siloxanes include, for example, polydimethylsiloxane, polydiethylsiloxane, and polymethylphenylsiloxane. Polydimethylsiloxane, which is also known as dimethicone, is especially preferred. These silicone compounds are available, for example, from the General Electric Company in their Viscasil^{®} and TSF 451 series, and from Dow Corning in their Dow Corning SH200 series.

The above polyalkylsiloxanes are available, for example, as a mixture with silicone compounds having a lower viscosity. Such mixtures have a viscosity of preferably from 1,000mPa·s to 100,000mPa·s, more preferably from 5,000mPa·s to 50,000mPa·s. Such mixtures preferably comprise: (i) a first silicone having a viscosity of from 100,000mPa·s to 30,000,000mPa·s at 25oC, preferably from 100,000mPa·s to 20,000,000mPa·s; and (ii) a second silicone having a viscosity of from 5mPa·s to 10,000mPa·s at 25oC, preferably from 5mPa·s to 5,000mPa·s. Such mixtures useful herein include, for example, a blend of dimethicone having a viscosity of 18,000,000mPa·s and dimethicone having a viscosity of 200mPa·s available from GE Toshiba, and a blend of dimethicone having a viscosity of 18,000,000mPa·s and cyclopentasiloxane available from GE Toshiba.

The silicone compounds useful herein also include a silicone gum. The term "silicone gum", as used herein, means a polyorganosiloxane material having a viscosity at 25°C of greater than or equal to 1,000,000 centistokes. It is recognized that the silicone gums described herein can also have some overlap with the above-disclosed silicone compounds. This overlap is not intended as a limitation on any of these materials. The "silicone gums" will typically have a mass molecular weight in excess of 200,000, generally between 200,000 and 1,000,000. Specific examples include polydimethylsiloxane, poly(dimethylsiloxane methylvinylsiloxane) copolymer, poly(dimethylsiloxane diphenylsiloxane methylvinylsiloxane) copolymer and mixtures thereof. The silicone gums are available, for example, as a mixture with silicone compounds having a lower viscosity. Such mixtures useful herein include, for example, Gum/Cyclomethicone blend available from Shin-Etsu.

Silicone compounds useful herein also include amino substituted materials. Preferred aminosilicones include, for example, those which conform to the general formula (I):

(R₁)ₐG₃₋ₐ-Si-(-OSiG₂)ₙ-(-OSiG_{b}(R₁)_{2-b})ₘ-O-SiG₃₋ₐ(R₁)ₐ

wherein G is hydrogen, phenyl, hydroxy, or C₁-C₈ alkyl, preferably methyl; a is 0 or an integer having a value from 1 to 3, preferably 1; b is 0, 1 or 2, preferably 1; n is a number from 0 to 1,999; m is an integer from 0 to 1,999; the sum of n and m is a number from 1 to 2,000; a and m are not both 0; R₁ is a monovalent radical conforming to the general formula CqH_{2q}L, wherein q is an integer having a value from 2 to 8 and L is selected from the following groups: -N(R₂)CH₂-CH₂-N(R₂)₂; -N(R₂)₂; -N(R₂)₃A ; -N(R₂)CH₂-CH₂-NR₂H₂A ; wherein R₂ is hydrogen, phenyl, benzyl, or a saturated hydrocarbon radical, preferably an alkyl radical from C₁ to C₂₀; A is a halide ion.

Highly preferred amino silicones are those corresponding to formula (I) wherein m=0, a=1, q=3, G=methyl, n is preferably from 1500 to 1700, more preferably 1600; and L is -N(CH₃)₂ or -NH₂, more preferably -NH₂. Another highly preferred amino silicones are those corresponding to formula (I) wherein m=0, a=1, q=3, G=methyl, n is preferably from 400 to 600, more preferably 500; and L is -N(CH₃)₂ or -NH₂, more preferably -NH₂. Such highly preferred amino silicones can be called as terminal aminosilicones, as one or both ends of the silicone chain are terminated by nitrogen containing group.

The above aminosilicones, when incorporated into the second composition, can be mixed with solvent having a lower viscosity. Such solvents include, for example, polar or non-polar, volatile or non-volatile oils. Such oils include, for example, silicone oils, hydrocarbons, and esters. Among such a variety of solvents, preferred are those selected from the group consisting of non-polar, volatile hydrocarbons, volatile cyclic silicones, non-volatile linear silicones, and mixtures thereof. The non-volatile linear silicones useful herein are those having a viscosity of from 1 to 20,000 centistokes, preferably from 20 to 10,000 centistokes at 25°C. Among the preferred solvents, highly preferred are non-polar, volatile hydrocarbons, especially non-polar, volatile isoparaffins, in view of reducing the viscosity of the aminosilicones and providing improved hair conditioning benefits such as reduced friction on dry hair. Such mixtures have a viscosity of preferably from 1,000mPa·s to 100,000mPa·s, more preferably from 5,000mPa·s to 50,000mPa·s.

Other suitable alkylamino substituted silicone compounds include those having alkylamino substitutions as pendant groups of a silicone backbone. Highly preferred are those known as "amodimethicone". Commercially available amodimethicones useful herein include, for example, BY16-872 available from Dow Corning.

The silicone compounds may further be incorporated in the second composition in the form of an emulsion, wherein the emulsion is made by mechanical mixing, or in the stage of synthesis through emulsion polymerization, with or without the aid of a surfactant selected from anionic surfactants, nonionic surfactants, cationic surfactants, and mixtures thereof.

### Additional Components

The second composition as described herein may include other additional components, which may be selected by the artisan according to the desired characteristics of the final product and which are suitable for rendering the second composition more cosmetically or aesthetically acceptable or to provide it with additional usage benefits. Such other additional components generally are used individually at levels of from 0.001% to 10%, preferably up to 5% by weight of the composition.

A wide variety of other additional components can be formulated into the present compositions. These include other conditioning agents such as: hydrolysed collagen with tradename Peptein 2000 available from Hormel, vitamin E with tradename Emix-d available from Eisai, panthenol available from Roche, panthenyl ethyl ether available from Roche, hydrolysed keratin, proteins, plant extracts, and nutrients; preservatives such as benzyl alcohol, methyl paraben, propyl paraben and imidazolidinyl urea; pH adjusting agents, such as citric acid, sodium citrate, succinic acid, phosphoric acid, sodium hydroxide, sodium carbonate; coloring agents, such as any of the FD&C or D&C dyes; perfumes; and sequestering agents, such as disodium ethylenediamine tetra-acetate; ultraviolet and infrared screening and absorbing agents such as benzophenones; and antidandruff agents such as zinc pyrithione and piroctone olamine.

### Method of Manufacturing

A method of preparing a personal care composition, wherein the first composition can be prepared as described in US20030103930A1, and the second composition can be prepared as described in US App. No. 13/617,240.

The cosmetic composition as described herein can be a multi-phase composition in that it comprises first and second compositions that are kept separate from one another until dispensed. Although kept separate until dispensing, the first and second composition are put into contact upon dispensing. The warming benefit may not occur until the cosmetic composition is dispensed and the first and second compositions interact. A variety of approaches may be used to dispense a multi-phase product. A common method is a multi-chamber tube or bottle. As used herein "The first and second compositions are kept separated from one another" means, for example, a package comprising two chambers, wherein the first composition is contained in a first chamber and the second composition is contained in a separate second chamber. Such packages can be shaped as a tube, pump, bottle or upside-down bottle, sachets and blister pack.

The first composition and the second composition may be not mixed before use. It is preferred that the first composition and the second composition may be mixed at a ratio of from 35:65 to 98:2, more preferably to 90: 10, still more preferably from 40:60 to 60:40.

While warming conditioners are known, current anhydrous warming conditioners are not necessarily able to deliver top conditioning benefits, such as superior silicone delivery through the use of gel networks or gel matrices. Similarly, some high performing conditioners lack the ability to offer a warming benefit without sacrificing their performance benefits. Therefore, it is possible to deliver high performance conditioning benefits while also providing a warming benefit. It has been discovered that certain ratios and viscosities of the first and second compositions described herein may provide a synergistic warming benefit compared to existing warming conditioners, while still providing excellent silicone deposition.

Warming conditioners using an anhydrous inorganic salt are not activated until mixed with water. Typically, conditioners using such warming agents rely on the water of the shower, for example, to provide the water necessary to start the warming. It has been discovered several things. First, an advantage may be had if the warming agent activation, that is, the triggering of heat that begins with the warming agent mixing with water, can happen in the user's hand, even before application in the hair and scalp. This allows for a superior warming benefit, as discussed below. But in order for the activation to occur in the hand, the water for activation must be provided in a form that is not as thin and runny as just water. That is, the warming agent must be mixed with a viscous-enough yet aqueous composition, such as the second composition as described herein. In addition, this more robust mixture is more easily transferred from the hand to the hair, allowing the heat to be felt sufficiently on the head, that is, not one that is quickly and easily washed away.

The warming benefit of the present composition is superior to that of either the first composition or the second composition alone. Even though the first composition comprises a warming agent, use of the first composition alone, while also not providing superior silicone deposition, also is not able to deliver the warming benefit of the multi-phase composition.

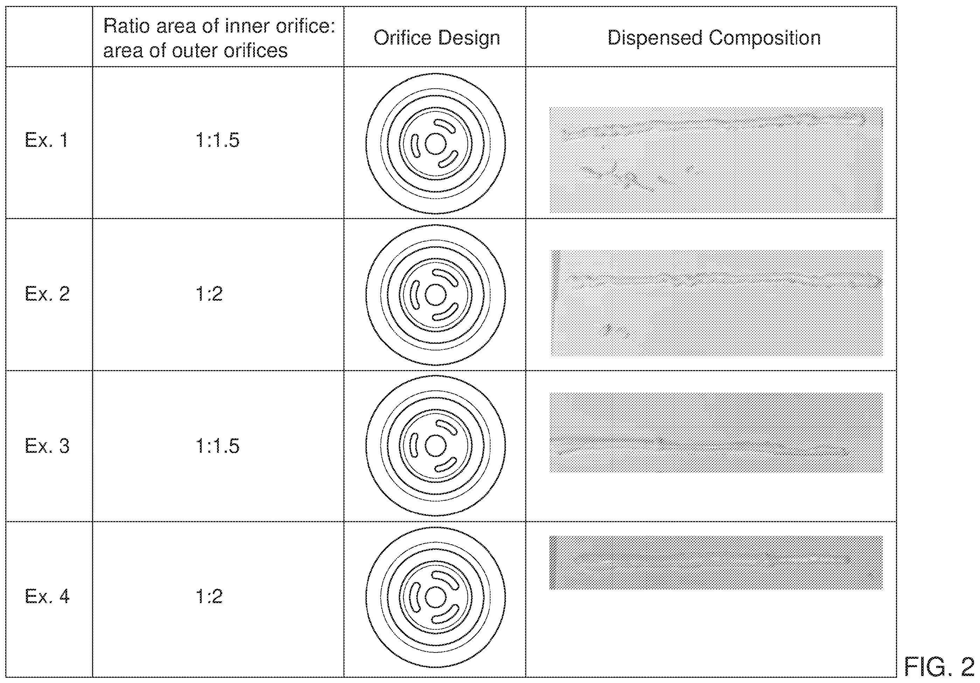

For example, Figure 1 is a graph of the maximum change in temperature a hair conditioner produces under the Maximum Temperature Change Test Method detailed below. The graph shows the maximum temperature change of various conditioners that are mixtures of the first and second compositions, specifically the compositions in Table 2. Table 1 is the data for Figures 1 and 2. The Figure 1 graph also shows, with the x mark on the far right, the maximum temperature change for a hair conditioner that is 100% of the first composition in Table 2, which is the composition that comprises an inorganic heat generating agent, in this case 15% anhydrous magnesium sulfate. Surprisingly, the conditioner that is 100% first composition does not provide the highest maximum temperature change. When both compositions are mixed, the total percentages of warming agent (magnesium sulfate) are reduced. Yet, with the reduced amount of warming agent, the inventive formulas have a greater maximum temperature change.

The conditioner that is 100% first composition provides 13 (12.63) degrees Celsius maximum temperature change or more, while the conditioner mixes that are 40%, 50%, 55%, 70%, 80%, 90%, 93%, 95%, and 98% of the first composition provide, respectively, 17.1, 17.4, 19.5, 25.4, 29.6, 29.1, 24.6, 20 and 14.6 degrees Celsius of maximum temperature change. As the Figure 1 graph shows, even as the percentage of the first composition example I comprising an inorganic heat generating agent is reduced, some combinations with the second composition, which does not comprise any heat generating agent, produce a higher maximum temperature change than the first composition alone. That is, even though the weight percentage of heat generating agent in the overall mixture is lower, a greater warming benefit results. When the level of inorganic heat generating agent is increased in first composition, more warming occurs and the maximum temperature change will subsequently increase.

One would expect that using a lower amount of magnesium sulfate would at least reduce the warming effect. However, the warming benefit of the multi-phase compositions is maintained and even is higher. The present inventors believe that the water in the second composition, delivered with a certain viscosity and in a certain ratio range with the first composition, allows for a synergistic boost to the magnesium sulfate's exothermic reaction.

Upon realizing this synergistic effect, the present inventors have isolated the particular ranges in which this effect exists. It is not simply a matter of combining a composition comprising a warming agent with some other aqueous composition, but becomes a matter of balancing the ratios of the first and second compositions, along with their respective viscosities in order to achieve the quick if not immediate mixing that allows the synergistic warming benefit to occur. Viscosity is a measure of how fluid the compositions are and thus how easily the compositions can be mixed together. Viscosities at a shear rate of 100 s⁻¹ are selected, as this low shear rate simulates the slow mixing of compositions on the palm by a consumer. As shown by the data, formulations that are outside certain ratios and viscosities do not produce the synergistic warming benefit, while those within the ratios and viscosities described herein exhibit the unexpected warming benefit. The viscosities between the first composition and the second composition may have a difference preferably less than 2.5 Pa.s and more preferably less than 2.0 Pa.s. It is believed that there is an upper limit to the difference in viscosities to exhibit the unexpected warming benefit. Beyond this upper limit, the viscosity difference is so great that it causes inhomogeneous mixing and thus delayed, less or no warming benefit at the specified ratios.

Figure 2 is a graph that plots the molar ratio of water to magnesium sulfate in the mixture versus the percent of the first composition in the mixture. The graph shows that the molar ratio of water to magnesium sulfate that produces the synergistic warming benefit may be from 75 to 0.75. The molar ratio of water in the second composition to the inorganic heating agent in the first composition may be from 75 to 0.75, which can then provide the unexpected warming benefit. If the molar ratio of water to inorganic heating agent is outside of this range, the warming benefit may not occur. The molar ratio that is effective can depend on the particular heating agent's enthalpy, wherein there may be an upper and lower limit to the molar ratio based on the heating agent's enthalpy. include, for example, chlorides such as calcium chloride (CaCl₂, CaCl₂.H₂O, CaCl₂.2H₂O), magnesium chloride (MgCl₂, MgCl₂.2 H₂O, MgCl₂.4 H₂O), aluminum chloride

### EXAMPLES

The following examples further describe and demonstrate aspects within the scope herein. Where applicable, ingredients are identified by chemical or CTFA name, or otherwise defined below.

**Table 1: Conditioning Composition**

| Component | Ex. A (wt. %) |
|---|---|
| BTMS/IPA *1 | 4.36 |
| Cetyl alcohol | 1.73 |
| Stearyl alcohol | 4.32 |
| Benzyl alcohol | 0.40 |
| Disodium EDTA | 0.13 |
| Polysorbate 20 | 0.075 |
| Citric acid | 0.04 |
| Silicone compound | 5.00 (*5) |
| Preservatives | 0.03 |
| Perfume | 1.07 |

| Deionized water | q.s. to 100% |
|---|---|
| Viscosity (Pa.s) | 0.57-0.73 |
| Yield Stress (Pa) | 1.12-3.05 |

**Table 2: Warming Composition**

| Components | Ex. B (wt. %) | Ex. C (wt. %) |
|---|---|---|
| BTAC/IPA *2 | 1.00 | 1.78 |
| CTAC/IPA *3 | 0.90 | - |
| Distearyl dimethyl ammonium Chloride | - | 0.8 |
| Cetyl alcohol | 1.00 | 0.5 |
| Stearyl alcohol | 1.80 | 1.3 |
| Silicone compound | 4.20 (*4) | - |
| Polyethylene/polypropylene block copolymer *6 | 1.00 | 3.00 |
| Polypropylene glycol *7 | 19.33 | 19.33 |
| Methyl bis-(oleyl amidoethyl) 2-hydroxyethyl ammonium methosulfate *8 | 2.00 | 2.00 |
| Anhydrous magnesium sulfate | 15.00 | 20.00 |
| Preservatives | 0.3 | 0.3 |
| Perfume | 0.65 | - |
| Polyethylene glycol *9 | q.s. to 100% | q.s. to 100% |
| Viscosity (Pa.s) | 1.88-2.36 | |
| Yield Stress (Pa) | 20.58-31.43 | |

### Definitions of Components

*1 BTMS/IPA: 80% of Behenyl Trimethyl Ammonium Methosulfate and 20% of Isopropyl alcohol
*2 BTAC/IPA: 80% of Behentrimonium Chloride and 20% of Isopropyl Alcohol
*3 CTAC/IPA: 50% of Cetrimonium Chloride and 50% of Isopropyl Alcohol
*4 Silicone compound: Dimethicone having a viscosity of 12,500 centistokes having a tradename Xiameter PMX-200 from Dow Corning.
*5 Silicone compound: Available from Momentive having a viscosity 10,000mPa·s, and having following formula (I):

   (R₁)ₐG₃₋ₐ-Si-(-OSiG₂)ₙ-(-OSiG_{b}(R₁)_{2-b})ₘ-O-SiG₃₋ₐ(R₁)ₐ (I)

   wherein G is methyl; a is an integer of 1; b is 0, 1 or 2, preferably 1; n is a number from 400 to 600; m is an integer of 0; R₁ is a monovalent radical conforming to the general formula CqH_{2q}L, wherein q is an integer of 3 and L is -NH₂
*6 Polyethylene/polypropylene block copolymer: Available from Sanyo^{®} Chemical with tradename of Newpol-108.
*7 Polypropylene Glycol: Available from Sanyo^{®} Chemicals with tradename of Newpol PPG-34 L or Newpol PPG-2000
*8 Methyl bis-(oleyl amidoethyl) 2-hydroxyethyl ammonium methosulfate: Available from Kao Chemicals with tradename of Tetranyl CO-40
*9 Polyethylene glycol: Available from BASF with tradename Pluracare E200

FIGS. 2, 3, 4A, and 4B and the accompanying text, below, shows the results from dispensing testing of different central orifice and outer orifice shapes and sizes in a tube-in-tube package with one tube containing the conditioning composition in Example A (*see* Table 1) and the other containing the warming composition in Example B (*see* Table 2). The dispensing test was done by squeezing the outer tube wall between a user's thumb and fingers 1/3 of the way up the tube, away from the nozzle (i.e. 2/3 of the way down from the sealed end of the tube).

It was important that the when the conditioning and warming compositions were dispensed the dual phase composition had a pleasing appearance that not only looked attractive in the user's hand but also distinctly showed both compositions, which provides a visual cue to guide the user to mix the composition well before application, thereby activating the heating mechanism and delivering the cleaning benefit.

The table in FIG. 2 shows the dispensed composition when dispensed through the orifice in Examples1-4. In Examples 1-4, the Example B (warming composition) was in the inner chamber and Example A (conditioning composition) was in the outer chamber. None of the dispensed compositions had the desired appearance, as shown in FIG. 2. In Examples 1-2, the conditioning composition that is dispensed through the outer orifices envelopes the warming composition that is dispensed through the central orifice. In these examples, the consumer could not see both compositions and would not intuitively know to mix the compositions in order to activate the warming effect and get a better conditioning effect.

Similarly, in Examples 3-4, which has 25% larger orifice area as compared to Examples 1-2, in some portions the conditioning composition envelopes the warming composition and in other areas the dispensed strand appears to be stripped and only the warming composition is seen. This appearance is not consumer preferred, as it is inconsistent and does not connotate a high-quality hair treatment composition. It is also believed that users may not intuitively mix compositions with this appearance.

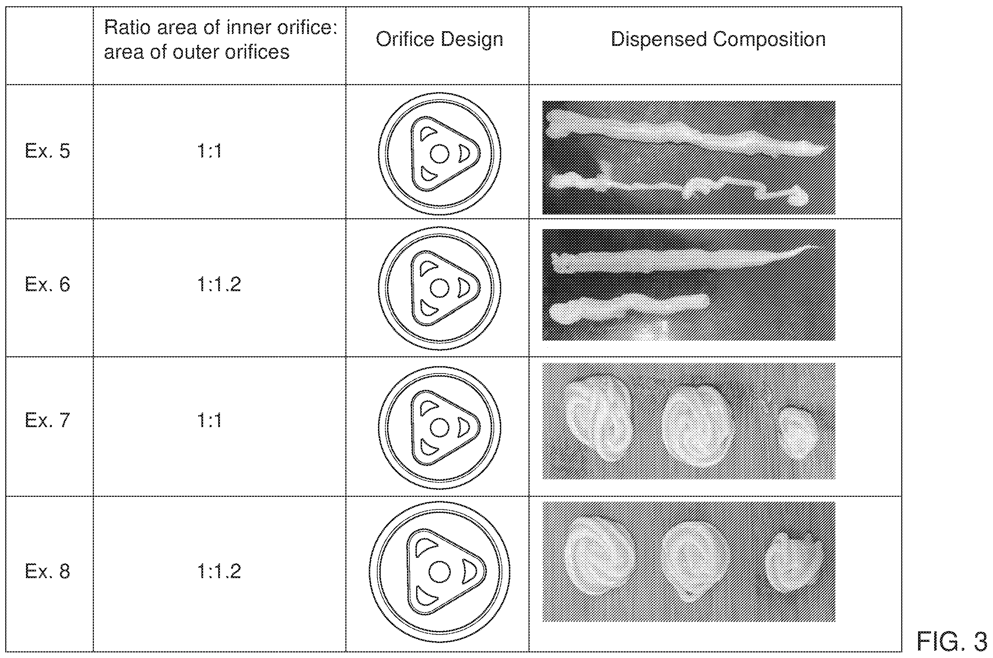

The table in FIG. 3 shows the dispensed composition when dispensed through the orifice in Examples 5-8. In Examples 5-6, Example B (warming composition, *see* Table 2) was in the inner chamber and Example A (conditioning composition, *see* Table 1) was in the outer chamber. Neither Example 5 nor 6 had a consumer acceptable appearance. Instead, a thick glob was dispensed, and it was difficult to differentiate the conditioning composition from the warming composition.

Examples 7-8 used the same orifices as Example 5-6, respectively. However, Example A (conditioning composition, *see* Table 1) was in the inner chamber and was dispensed through the central orifice and Example B (warming composition, *see* Table 2) was in the outer chamber and dispensed through the outer orifices. Both strands dispensed in Examples 7 and 8 had a consumer preferred attractive appearance. As shown in FIG. 3, the dispensed composition for Examples 7-8 has a uniform appearance with smooth strands and both compositions are visible throughout. It was found that when a consumer dispensed a composition that had an appearance like Examples 7 or 8, they would instinctively mix the composition, thereby further activating the warming sensation. In some instances, Examples 7-8 can be less preferred because it can be difficult to fill the chambers with the conditioning composition in the outer chamber and the warming composition in the inner chamber.

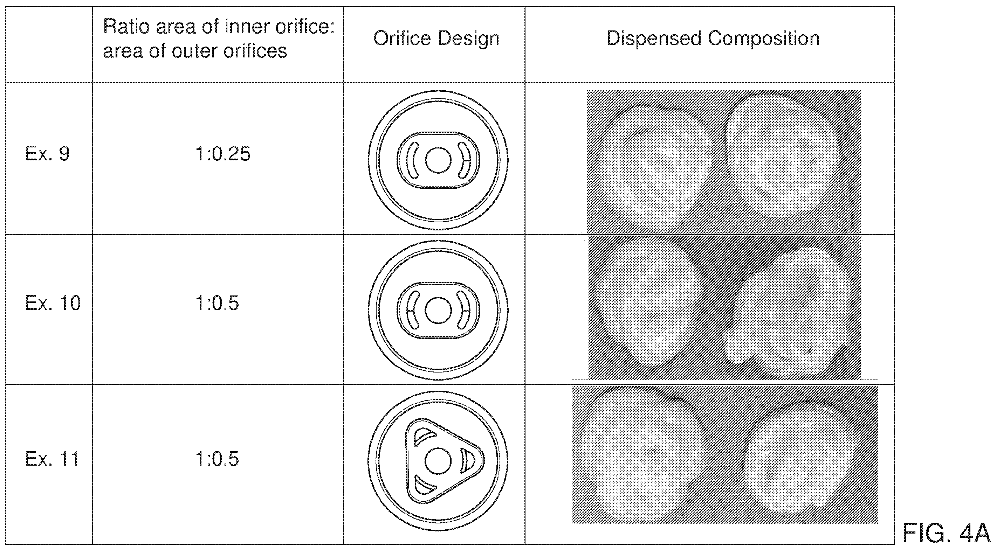

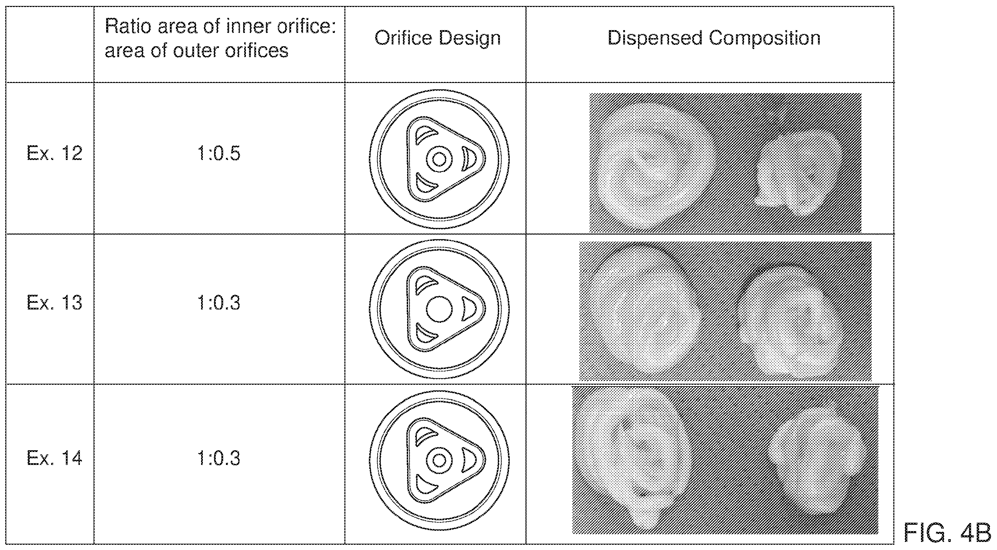

The tables in FIGS. 4A and 4B show the dispensed composition when dispensed through the orifice in Examples 9-14. In Examples 9-14, the Example B (warming composition, *see* Table 2) was in the inner chamber and Example A (conditioning composition, *see* Table 1) was in the outer chamber. Examples 9-14 all have a dispensed composition where both the warming composition and the conditioner composition are visible, which may prompt the consumer to mix the composition to further activate the warming.

The appearance in Examples 9 and 10 may be consumer preferred for the following reasons. First, it was found that the ratio of the area of the inner orifice to the area of the outer orifice of 1:0.25 (4:1) provides the most even dispensing of the conditioning composition and the warming composition from the first to the last use. Second, it was determined that one larger outer orifice can be preferred over multiple (e.g. 2 or 3) smaller orifices because multiple outer orifices caused the dispensed strand to have a noodle like appearance that is not aesthetically pleasing. Further, smaller orifices tend to dispense at a higher pressure that can cause the conditioner composition to detach from the warming composition, instead of dispensing as a unified strand. Third, it was found that a slot shape, in particular a curved slot shape, can be preferred over a round circular or oval shape, since the round shape can also dispense like a noodle and the warming phase and the conditioner phase can be detached.

## Claims

1. A rinse-off hair treatment product comprising:
a. a tube package (1) comprising:
i. an inner tube (4) comprising an inner tube wall (44) and an inner chamber (41);
ii. an outer tube (3) comprising an outer tube wall (34) an outer chamber (31) formed between the outer tube wall (44) and the inner tube wall (34);
iii. one or more outer orifices (53) fluidly connected to the outer chamber (31) by one or more outer nozzle channels (33);
iv. one or more central orifices (54) fluidly connected to the inner chamber (41) by one or more inner nozzle channels (43);
wherein the ratio of the area of the one or more central orifices (54) to the area of the one or more outer orifices (53) is from 2 to 6, preferably from 2.5 to 5.5, more preferably from 3 to 5, and even more preferably from 3.5 to 4.5;
b. a dual-phase hair treatment composition comprising:
i. a warming composition (46) comprising a viscosity;
ii. a conditioning composition (36) comprising a viscosity;
wherein the viscosity of the warming composition (46) is greater than the viscosity of the conditioning composition (36);
wherein inner chamber (41) contains the warming composition (46);
wherein the outer chamber (31) contains the conditioning composition (36);
wherein the warming composition (46) and the conditioning composition (36) are physically separated within the tube package (1).

2. The product according to claim 1, wherein the viscosity of the warming composition is at least 1.25 times larger than the viscosity of the conditioning composition, preferably at least 1.4 times, and more preferably 1.5 times.

3. The product according to any preceding claims, wherein a weight ratio of the conditioning composition to the warming composition is from 1 to 5, preferably from 1.25 to 2.25, more preferably from 1.35 to 1.9, and even more preferably from 1.5 to 1.75.

4. The product according to any preceding claims, wherein the one or more central orifices (54) comprises one orifice and the one or more outer orifices (53) comprises one orifice.

5. The product according to claim 5, wherein the inner orifice (54) is a circle or oval.

6. The product of according to claims 5-6, wherein the outer orifice (53) is a slit, preferably a curved slit.

7. The product of according to paragraphs 5-7, wherein the inner orifice (54) has an area from 2 mm² to 9 mm², preferably from 3 mm² to 7 mm², more preferably from 4 mm² to 6 mm², and even more preferably from 4.5 mm² to 5.5 mm² and wherein the outer orifice (53) has an area from 0.25 mm² to 5 mm², preferably from 0.5 mm² to 3.5 mm², more preferably from 0.75 mm² to 2.5 mm², and even more preferably from 1 mm² to 1.5 mm².

8. The product of according to any preceding claims, wherein the outer tube wall (34) and the inner tube (44) wall comprise a laminated film comprising a polymer and an aluminum barrier.

9. The product of according to any preceding claims, wherein the conditioning composition (36) and the warming composition (46) are different colors.

10. The product of according to any preceding claims, wherein the warming composition (46) comprises an inorganic heat generating agent that generates a heat by mixing with water and the conditioning composition comprises a cationic surfactant system comprising a mono-alkyl quaternized ammonium salt cationic surfactant, a high melting point fatty compound, and an aqueous carrier.

11. The product according to claim 10, wherein the inorganic heat generating agent is an anhydrous inorganic salt selected from the group consisting of calcium sulfate, magnesium sulfate, aluminum sulfate, calcium chloride, magnesium chloride, calcium oxide, and mixtures thereof.

12. The product of according to any preceding claims, wherein the warming composition (46) comprises a gel matrix comprising at least a portion of the cationic surfactant, the high melting point fatty compound, and the aqueous carrier.

13. A method of treating hair comprising:
a. providing the rinse-off hair treatment product according to any preceding claims;
b. applying pressure to the outer tube wall (34) and dispensing the dual-phase hair treatment composition as a single strand into a user's palm;
c. mixing the dual-phase hair treatment composition thereby promoting a warming sensation;
d. applying the dual-phase hair treatment composition to a user's hair and/or scalp;
e. rinsing the dual-phase hair treatment composition from the user's hair and/or scalp.

14. The method of claim 13, wherein the weight ratio of the dispensed conditioner composition to the dispensed warming composition in the strand is from 1 to 3, preferably from 1.1 to 2, and more preferably from 1.3 to 1.5.

## Patentansprüche

1. Abspül-Haarbehandlungsprodukt, umfassend:
a. eine Röhrenverpackung (1), umfassend:
i. eine innere Röhre (4), umfassend eine innere Röhrenwand (44) und eine innere Kammer (41);
ii. eine äußere Röhre (3), umfassend eine äußere Röhrenwand (34) und eine äußere Kammer (31), die zwischen der äußeren Röhrenwand (44) und der inneren Röhrenwand (34) ausgebildet ist;
iii. eine oder mehrere äußere Öffnungen (53), die mit der äußeren Kammer (31) durch einen oder mehrere äußere Düsenkanäle (33) fluidisch verbunden sind;
iv. eine oder mehrere zentrale Öffnungen (54), die mit der inneren Kammer (41) durch einen oder mehrere innere Düsenkanäle (43) fluidisch verbunden sind;
wobei das Verhältnis der Fläche der einen oder der mehreren zentralen Öffnungen (54) zu der Fläche der einen oder der mehreren äußeren Öffnungen (53) von 2 bis 6, vorzugsweise von 2,5 bis 5,5, mehr bevorzugt von 3 bis 5 und noch mehr bevorzugt von 3,5 bis 4,5, beträgt;
b. eine zweiphasige Haarbehandlungszusammensetzung, umfassend:
i. eine Wärmezusammensetzung (46), umfassend eine Viskosität;
ii. eine Konditionierungszusammensetzung (36), umfassend eine Viskosität;
wobei die Viskosität der Wärmezusammensetzung (46) größer als die Viskosität der Konditionierungszusammensetzung (36) ist;
wobei die innere Kammer (41) die Wärmezusammensetzung (46) enthält;
wobei die äußere Kammer (31) die Konditionierungszusammensetzung (36) enthält;
wobei die Wärmezusammensetzung (46) und die Konditionierungszusammensetzung (36) innerhalb der Röhrenverpackung (1) physisch getrennt sind.

2. Produkt nach Anspruch 1, wobei die Viskosität der Wärmezusammensetzung wenigstens 1,25-mal größer als die Viskosität der Konditionierungszusammensetzung ist, vorzugsweise wenigstens 1,4-mal und mehr bevorzugt 1,5-mal.

3. Produkt nach einem der vorstehenden Ansprüche, wobei ein Gewichtsverhältnis der Konditionierungszusammensetzung zu der Wärmezusammensetzung von 1 bis 5, vorzugsweise von 1,25 bis 2,25, mehr bevorzugt von 1,35 bis 1,9 und noch mehr bevorzugt von 1,5 bis 1,75, beträgt.

4. Produkt nach einem der vorstehenden Ansprüche, wobei die eine oder die mehreren zentralen Öffnungen (54) eine Öffnung umfassen und die eine oder die mehreren äußeren Öffnungen (53) eine Öffnung umfassen.

5. Produkt nach Anspruch 5, wobei die innere Öffnung (54) ein Kreis oder ein Oval ist.

6. Produkt nach den Ansprüchen 5 bis 6, wobei die äußere Öffnung (53) ein Schlitz, vorzugsweise ein gekrümmter Schlitz, ist.

7. Produkt nach den Absätzen 5 bis 7, wobei die innere Öffnung (54) eine Fläche von 2 mm² bis 9 mm², vorzugsweise von 3 mm² bis 7 mm², mehr bevorzugt von 4 mm² bis 6 mm² und noch mehr bevorzugt von 4,5 mm² bis 5,5 mm², aufweist und wobei die äußere Öffnung (53) eine Fläche von 0,25 mm² bis 5 mm², vorzugsweise von 0,5 mm² bis 3,5 mm², mehr bevorzugt von 0,75 mm² bis 2,5 mm² und noch mehr bevorzugt von 1 mm² bis 1,5 mm², aufweist.

8. Produkt nach einem der vorstehenden Ansprüche, wobei die äußere Röhrenwand (34) und die Wand der inneren Röhre (44) eine laminierte Folie umfassen, umfassend ein Polymer und eine Aluminiumsperre.

9. Produkt nach einem der vorstehenden Ansprüche, wobei die Konditionierungszusammensetzung (36) und die Wärmezusammensetzung (46) von unterschiedlichen Farben sind.

10. Produkt nach einem der vorstehenden Ansprüche, wobei die Wärmezusammensetzung (46) ein anorganisches wärmeerzeugendes Mittel umfasst, das durch ein Mischen mit Wasser eine Wärme erzeugt, und die Konditionierungszusammensetzung ein kationenaktives Tensidsystem umfasst, umfassend ein kationenaktives quaternisiertes Monoalkylammoniumsalztensid, eine Fettverbindung mit hohem Schmelzpunkt und einen wässrigen Träger.

11. Produkt nach Anspruch 10, wobei das anorganische, wärmeerzeugende Mittel ein wasserfreies anorganisches Salz ist, das ausgewählt ist aus der Gruppe, bestehend aus Kalziumsulfat, Magnesiumsulfat, Aluminiumsulfat, Kalziumchlorid, Magnesiumchlorid, Kalziumoxid und Mischungen davon.

12. Produkt nach einem der vorstehenden Ansprüche, wobei die Wärmezusammensetzung (46) eine Gelmatrix umfasst, umfassend wenigstens einen Teil des kationenaktiven Tensids, der Fettverbindung mit hohem Schmelzpunkt und des wässrigen Trägers.

13. Verfahren zum Behandeln von Haar, umfassend:
a. Bereitstellen des Abspül-Haarbehandlungsprodukts nach einem der vorstehenden Ansprüche;
b. Ausüben von Druck auf die äußere Röhrenwand (34) und Abgeben der zweiphasigen Haarbehandlungszusammensetzung als ein einzelner Strang in die Handfläche eines Benutzers;
c. Mischen der zweiphasigen Haarbehandlungszusammensetzung, wobei dadurch ein Wärmegefühl gefördert wird;
d. Aufbringen der zweiphasigen Haarbehandlungszusammensetzung auf das Haar und/oder die Kopfhaut eines Benutzers;
e. Abspülen der zweiphasigen Haarbehandlungszusammensetzung von dem Haar und/oder der Kopfhaut des Benutzers.

14. Verfahren nach Anspruch 13, wobei das Gewichtsverhältnis der abgegebenen Conditioner-Zusammensetzung zu der abgegebenen Wärmezusammensetzung in dem Strang von 1 bis 3, vorzugsweise von 1,1 bis 2 und mehr bevorzugt von 1,3 bis 1,5, beträgt.

## Revendications

1. Produit de traitement de cheveux à éliminer par rinçage, comprenant :
a. un emballage en tube (1) comprenant :
i. un tube interne (4) comprenant une paroi de tube interne (44) et une chambre interne (41) ;
ii. un tube externe (3) comprenant une paroi de tube externe (34), une chambre externe (31) formée entre la paroi de tube externe (44) et la paroi de tube interne (34) ;
iii. un ou plusieurs orifices externes (53) reliés fluidiquement à la chambre externe (31) par un ou plusieurs canaux de buse externe (33) ;
iv. un ou plusieurs orifices centraux (54) reliés fluidiquement à la chambre interne (41) par un ou plusieurs canaux de buse interne (43) ;
dans lequel le rapport de la zone de l'orifice ou des orifices centraux (54) à la zone de l'orifice ou des orifices externes (53) va de 2 à 6, de préférence de 2,5 à 5,5, plus préférablement de 3 à 5, et encore plus préférablement de 3,5 à 4,5 ;
b. une composition de traitement de cheveux à double phase, comprenant :
i. une composition chauffante (46) comprenant une viscosité ;
ii. une composition de conditionnement (36) comprenant une viscosité ;
dans lequel la viscosité de la composition de chauffante (46) est supérieure à la viscosité de la composition de conditionnement (36) ;
dans lequel la chambre interne (41) contient la composition chauffante (46) ;
dans lequel la chambre externe (31) contient la composition de conditionnement (36) ;
dans lequel la composition chauffante (46) et la composition de conditionnement (36) sont physiquement séparées à l'intérieur de l'emballage en tube (1).

2. Produit selon la revendication 1, dans lequel la viscosité de la composition chauffante est au moins 1,25 fois supérieure à la viscosité de la composition de conditionnement, de préférence au moins 1,4 fois, et plus préférablement 1,5 fois.

3. Produit selon l'une quelconque des revendications précédentes, dans lequel un rapport pondéral entre la composition de conditionnement et la composition chauffante va de 1 à 5, de préférence de 1,25 à 2,25, plus préférablement de 1,35 à 1,9, et encore plus préférablement de 1,5 à 1,75.

4. Produit selon l'une quelconque des revendications précédentes, dans lequel l'orifice ou les orifices centraux (54) comprennent un orifice et l'orifice ou les orifices externes (53) comprennent un orifice.

5. Produit selon la revendication 5, dans lequel l'orifice interne (54) est un cercle ou un ovale.

6. Produit selon les revendications 5 à 6, dans lequel l'orifice externe (53) est une fente, de préférence une fente incurvée.

7. Produit selon les paragraphes 5 à 7, dans lequel l'orifice interne (54) couvre une zone allant de 2 mm² à 9 mm², de préférence de 3 mm² à 7 mm², plus préférablement de 4 mm² à 6 mm², et encore plus préférablement de 4,5 mm² à 5,5 mm², et dans lequel l'orifice externe (53) couvre une zone allant de 0,25 mm² à 5 mm², de préférence de 0,5 mm² à 3,5 mm², plus préférablement de 0,75 mm² à 2,5 mm², et encore plus préférablement de 1 mm² à 1,5 mm².

8. Produit selon l'une quelconque des revendications précédentes, dans lequel la paroi du tube externe (34) et la paroi du tube interne (44) comprennent un film stratifié comprenant un polymère et une barrière en aluminium.

9. Produit selon l'une quelconque des revendications précédentes, dans lequel la composition de conditionnement (36) et la composition chauffante (46) sont de couleurs différentes.

10. Produit selon l'une quelconque des revendications précédentes, dans lequel la composition chauffante (46) comprend un agent générateur de chaleur inorganique qui génère une chaleur en se mélangeant à de l'eau, et la composition de conditionnement comprend un système tensioactif cationique comprenant un agent tensioactif cationique de type sel d'ammonium quaternaire mono-alkyle, un composé gras à haut point de fusion et un véhicule aqueux.

11. Produit selon la revendication 10, dans lequel l'agent de génération de chaleur inorganique est un sel inorganique anhydre choisi dans le groupe constitué de sulfate de calcium, sulfate de magnésium, sulfate d'aluminium, chlorure de calcium, chlorure de magnésium, oxyde de calcium, et mélanges de ceux-ci.

12. Produit selon l'une quelconque des revendications précédentes, dans lequel la composition chauffante (46) comprend une matrice de gel comprenant au moins une partie de l'agent tensioactif cationique, du composé gras à haut point de fusion et du véhicule aqueux.

13. Procédé de traitement de cheveux comprenant :
a. la fourniture du produit de traitement de cheveux à éliminer par rinçage selon l'une quelconque des revendications précédentes ;
b. l'application d'une pression sur la paroi externe de tube (34) et la distribution de la composition de traitement de cheveux à double phase sous la forme d'un fil unique dans la paume d'un utilisateur ;
c. le mélange de la composition de traitement de cheveux à double phase, favorisant de ce fait une sensation de chaleur ;
d. l'application de la composition de traitement de cheveux à double phase sur les cheveux et/ou le cuir chevelu d'un utilisateur ;
e. le rinçage de la composition de traitement de cheveux à double phase des cheveux et/ou du cuir chevelu de l'utilisateur.

14. Procédé selon la revendication 13, dans lequel le rapport en poids de la composition de conditionnement distribuée à la composition chauffante distribuée dans le fil va de 1 à 3, de préférence de 1,1 à 2, et plus préférablement de 1,3 à 1,5.
